(19) **Europäisches Patentamt**
**European Patent Office**
**Office européen des brevets**

(11) **EP 4 139 666 B1**

(12) **EUROPÄISCHE PATENTSCHRIFT**

(45) Veröffentlichungstag und Bekanntmachung des Hinweises auf die Patenterteilung:
**20.11.2024 Patentblatt 2024/47**

(21) Anmeldenummer: **21719094.1**

(22) Anmeldetag: **13.04.2021**

(51) Internationale Patentklassifikation (IPC):
**G01N 23/02** *(2006.01)* **G01T 1/24** *(2006.01)*

(52) Gemeinsame Patentklassifikation (CPC):
**G01N 23/02; A61B 6/032; A61B 6/4258; A61B 6/5205; G01T 1/2985**

(86) Internationale Anmeldenummer:
**PCT/EP2021/059483**

(87) Internationale Veröffentlichungsnummer:
**WO 2021/213837 (28.10.2021 Gazette 2021/43)**

(54) **VORRICHTUNG UND VERFAHREN ZUR TRANSMISSIONSBILDGEBUNG**

TRANSMISSION IMAGING DEVICE AND METHOD

DISPOSITIF ET PROCÉDÉ D'IMAGERIE DE TRANSMISSION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.04.2020 DE 102020111182**

(43) Veröffentlichungstag der Anmeldung:
**01.03.2023 Patentblatt 2023/09**

(73) Patentinhaber: **Helmholtz-Zentrum Dresden - Rossendorf e.V.**
**01328 Dresden (DE)**

(72) Erfinder: **BARTHEL, Frank**
**01328 Dresden (DE)**

(56) Entgegenhaltungen:
**WO-A2-2004/095060**   **US-A- 5 818 898**
**US-A1- 2006 008 047**   **US-A1- 2014 376 695**
**US-A1- 2020 046 310**

- **BIEBERLE MARTINA ET AL: "Ultrafast X-ray computed tomography for the analysis of gas-solid fluidized beds", CHEMICAL ENGENEERING JOURNAL, vol. 189-190, May 2012 (2012-05-01), AMSTERDAM, NL, pages 356 - 363, XP055814676, ISSN: 1385-8947, DOI: 10.1016/j.cej.2012.02.028**

**Beschreibung**

Die Erfindung betrifft eine Vorrichtung und ein Verfahren zur Transmissionsbildgebung, z.B. zur Bildgebung mittels Röntgenstrahlung oder mittels Gammastrahlung, mittels derer eine Abbildung des Inneren eines Untersuchungsobjekts ermöglicht ist.

Bei der Transmissionsbildgebung wird ein zu untersuchendes Objekt, z.B. ein Patient oder ein Prüfobjekt, mit hochenergetischer Strahlung durchleuchtet bzw. durchstrahlt. Die durch das Untersuchungsobjekt hindurchtretende und von diesem geschwächte Strahlung wird mittels Strahlungsdetektoren erfasst, wobei anhand der Detektormesswerte eine Abbildung des Inneren des Untersuchungsobjekts generiert werden kann. So wird z.B. bei der herkömmlichen Röntgenbildgebung eine Projektionsabbildung des Inneren eines Untersuchungsobjekts erzeugt, indem das Untersuchungsobjekt entlang einer vorgegebenen Richtung mit Röntgenstrahlung durchstrahlt wird und mittels einer im Strahlengang hinter dem Objekt angeordneten röntgensensitiven Detektoranordnung die Röntgenstrahlung nach Wechselwirkung mit dem Objekt detektiert wird. Als ein anderes Beispiel werden bei der Röntgentomographie Schnittbilder eines Untersuchungsobjekts aufgenommen, indem die Röntgenstrahlungsquelle und der Röntgendetektor relativ zueinander bewegt werden, wobei überlagerungsfreie Darstellungen einer Schicht des Untersuchungsobjekts gewonnen werden können.

Bei der Röntgen-Computertomographie wird das abzubildende Untersuchungsobjekt aus unterschiedlichen Richtungen mit Röntgenstrahlung durchleuchtet. Die durch das Untersuchungsobjekt hindurchtretende und von diesem geschwächte Röntgenstrahlung wird mittels mehrerer Röntgendetektoren erfasst, z.B. mittels eines kreis- oder teilkreisförmigen Röntgendetektorbogens aus mehreren Röntgendetektoren. Es werden Strahlschwächungsprofile bei Durchstrahlung des Untersuchungsobjekts aus unterschiedlichen Richtungen erstellt und aus den Messdaten durch Anwendung tomographischer Bildrekonstruktionsverfahren die Dichteverteilung in der durchstrahlten Schnittebene berechnet. Die Röntgen-Computertomographie wird z.B. in der medizinischen Diagnostik (z.B. zur Bildgebung des schlagenden Herzens) und in der Prozesstomographie (z.B. zur Strömungsdiagnostik oder Materialprüfung) eingesetzt.

Eine Transmissionsbildgebung kann mittels kontinuierlicher Strahlung oder mittels gepulster Strahlung erfolgen. Bei der Anwendung gepulster Strahlung wird ein zu untersuchendes Objekt mit Pulsen elektromagnetischer Strahlung durchstrahlt, wobei die Pulse Pulsdauern von wenigen Nanosekunden bis zu mehreren Sekunden aufweisen können. Mittels eines aus mehreren separaten Strahlungsdetektoren bestehenden Detektorarrays kann die innerhalb eines vorgegebenen Mess-Zeitfensters auf das Detektorarray auftreffende Strahlung detektiert und daraus eine Abbildung des Inneren des durchstrahlten Untersuchungsobjekts generiert werden. Die Verwendung desselben Mess-Zeitfensters für alle Strahlungsdetektoren kann zu Verfälschungen und Qualitätsverlusten der erzeugten Abbildungen führen. Die Verwendung desselben Mess-Zeitfensters für alle Strahlungsdetektoren bedeutet, dass für alle Strahlungsdetektoren des Detektorarrays die Strahlungsdetektion zu demselben Zeitpunkt begonnen wird und zu demselben Zeitpunkt beendet wird, wobei der Anfangszeitpunkt eines solchen Mess-Zeitfensters auch als Detektions-Anfangszeitpunkt und der Endzeitpunkt eines solchen Mess-Zeitfensters auch als Detektions-Endzeitpunkt bezeichnet wird. Ein derartiges Mess-Zeitfenster wird auch kurz als Messfenster bezeichnet.

Das zur Strahlungsdetektion verwendete Detektorarray kann z.B. Detektoren mit unterschiedlichen Abständen zu der zur Erzeugung der Strahlungspulse verwendeten Strahlungsquelle aufweisen, z.B. bei Verwendung einer punktförmigen Strahlungsquelle in Verbindung mit einem ebenen Detektorarray. In diesem Fall ist die Laufzeit, welche die Pulse zum Erreichen der näher an der Strahlungsquelle angeordneten Detektoren benötigen, kleiner als die Laufzeit, welche die Pulse zum Erreichen von weiter von der Strahlungsquelle weg angeordneten Detektoren benötigen. Die Verwendung desselben Mess-Zeitfensters, insbesondere desselben Detektions-Endzeitpunktes, für alle Detektoren kann z.B. dazu führen, dass ein Puls, welcher von einem näher an der Strahlungsquelle angeordneten Detektor innerhalb eines Mess-Zeitfensters noch vollständig erfasst wird, von einem (im Vergleich dazu) weiter von der Strahlungsquelle entfernten Detektor aufgrund der längeren Laufzeit innerhalb desselben Mess-Zeitfensters nur noch teilweise erfasst wird. Zudem kann die zum Durchstrahlen verwendete Strahlung im Untersuchungsobjekt unter Richtungsänderung ein- oder mehrmals gestreut werden, wobei derart gestreute Strahlung einen längeren Weg bis zum Erreichen eines jeweiligen Detektors zurücklegt als ungestreute Strahlung, sodass derart gestreute Strahlung auch eine längere Laufzeit bis zum Erreichen eines jeweiligen Detektors benötigt als ungestreute Strahlung. Der auf derartige gestreute Strahlung zurückgehende Anteil eines Detektorsignals wird auch als Streustrahlungsanteil des Detektorsignals bezeichnet. Die Verwendung desselben Mess-Zeitfensters, insbesondere desselben Detektions-Endzeitpunktes, für alle Detektoren kann z.B. auch dazu führen, dass für unterschiedlich weit von der Strahlungsquelle entfernte Detektoren unterschiedliche Streustrahlungsanteile der jeweiligen Detektorsignale vorliegen. Die Verwendung desselben Mess-Zeitfensters, insbesondere desselben Detektions-Endzeitpunktes, für alle Detektoren kann z.B. dazu führen, dass auf den ungestreuten Pulsanteil nachfolgende gestreute Strahlung an einem weiter von der Strahlungsquelle entfernten Detektor im Wesentlichen erst nach dem Detektions-Endzeitpunkt eintrifft und somit hier nicht mehr oder nur zu einem kleinen Teil erfasst wird, wohingegen an einem näher an der Strahlungsquelle angeordneten Detektor auch auf den ungestreuten Pulsanteil nachfolgende gestreute Strahlung noch vor dem Detektions-Endzeitpunkt eintrifft und somit hier zu einem größeren Teil erfasst wird, sodass die beiden Detektorsignale unterschiedliche Streustrahlungsanteile aufweisen. Da der Streustrahlungsanteil sich in einem Gleichanteil bzw. Grundgrauwert in der erzeugten Abbildung äußert, können unterschiedliche Streustrahlungsanteile der einzelnen Detektorsignale zu unterschiedlichen Gleichanteilen und somit einem ortsabhängig variierenden Grundgrauwert in der erzeugten Abbildung führen, wodurch die erzeugte Abbildung verfälscht werden kann.

Herkömmliche, auf der einheitlichen Ansteuerung aller Strahlungsdetektoren basierende Transmissionsbildgebungen mittels gepulster Strahlung sind somit nur eingeschränkt zum hochqualitativen und unverfälschten Abbilden des Inneren von Untersuchungsobjekten geeignet.

Die US 2006/008047 A1 beschreibt ein Computertomographiegerät und entsprechende Computertomographieverfahren, wobei das Computertomographiegerät eine Röntgenquelle und einen Röntgendetektor aufweist, und wobei die Röntgenquelle eine Kathode mit mehreren individuell programmierbaren Elektronenemissionseinheiten und eine Anode zum Emittieren von Röntgenstrahlung beim Auftreffen von Elektronen darauf aufweist.

Durch die Erfindung werden ein Verfahren und eine Vorrichtung zur Transmissionsbildgebung mittels gepulster Strahlung bereitgestellt, mittels derer auf einfache Art und Weise ein hochqualitatives Abbilden des Inneren eines Untersuchungsobjekts ermöglicht ist.

Gemäß einem ersten Aspekt der Erfindung wird eine Vorrichtung zur Transmissionsbildgebung zum Abbilden des Inneren eines Untersuchungsobjekts bereitgestellt, wobei die Vorrichtung auch als Abbildungsvorrichtung bezeichnet wird. Die Abbildungsvorrichtung kann eine Aufnahme (auch als Aufnahmeraum bezeichnet) zum Aufnehmen eines Untersuchungsobjekts darin aufweisen, das abzubildende Untersuchungsobjekt wird demgemäß in der Aufnahme positioniert. Die Aufnahme kann z.B. durch einen Aufnahmebereich gegeben sein, in dem das abzubildende Untersuchungsobjekt während der Bilderzeugung positioniert wird.

Die Abbildungsvorrichtung weist eine Vorrichtung zum Erzeugen von Pulsen elektromagnetischer Strahlung auf, wobei diese Vorrichtung auch als Strahlungserzeugungsvorrichtung oder Strahlungserzeuger bezeichnet wird. Die Strahlung wird zum Durchstrahlen des Untersuchungsobjekts zur Transmissionsbildgebung verwendet. Bei der Strahlung kann es sich z.B. um Röntgenstrahlung oder Gammastrahlung handeln. Da die Strahlung zum Untersuchen des Untersuchungsobjekts verwendet wird, wird die Strahlung auch als Untersuchungsstrahlung bezeichnet. Die Strahlungserzeugungsvorrichtung ist zum Emittieren bzw. Abstrahlen von Pulsen elektromagnetischer Strahlung (auch als Strahlungspulse bezeichnet) ausgehend von einer Strahlungsquellposition ausgebildet. Die Strahlungspulse verlaufen von der Strahlungsquellposition ausgehend, wobei die Strahlungspulse z.B. erst an der Strahlungsquellposition erzeugt werden können oder an einer anderen Position zu der Strahlungsquellposition hin verlaufend erzeugt werden können und anschließend von der Strahlungsquellposition ausgehend verlaufen. Der Strahlungserzeuger kann z.B. eine laserbasierte oder elektronenstrahlbasierte Strahlungsquelle (insbesondere Röntgenstrahlungsquelle) sein, bei der mittels Materialanregung mittels eines gepulsten Laser- bzw. Elektronenstrahls gepulste Untersuchungsstrahlung (insbesondere Röntgenstrahlung) generiert wird. Die Strahlungserzeugungsvorrichtung kann z.B. eine Röntgenbremsstrahlungsquelle sein.

Der Strahlungserzeuger kann z.B. zum Abstrahlen von Strahlungspulsen ausgehend von einer punktförmigen Strahlungsquellposition ausgebildet ist. Des Weiteren kann z.B. vorgesehen sein, dass der Strahlungserzeuger zum Emittieren von divergent von einer punktförmigen Strahlungsquellposition ausgehenden Strahlungspulsen ausgebildet ist, sodass die Strahlungspulse z.B. kegelförmig von der Strahlungsquellposition ausgehen.

Die erzeugte pulsförmige Untersuchungsstrahlung verläuft in Richtung zu dem Aufnahmeraum hin, sodass bei in dem Aufnahmeraum angeordnetem Untersuchungsobjekt die Strahlung zu dem Untersuchungsobjekt hin verläuft und dieses unter Wechselwirkung (z.B. Absorption, Streuung) durchstrahlt. Der Aufnahmeraum bzw. ein darin aufgenommenes Untersuchungsobjekt wird somit von der gepulsten Strahlung durchstrahlt. Beim Durchstrahlen des Untersuchungsobjekts wechselwirkt die Strahlung mit dem Untersuchungsobjekt und wird dabei modifiziert, wobei die transmittierte Strahlung Rückschlüsse auf die innere Struktur des Untersuchungsobjekts ermöglicht. Die durch die Wechselwirkung mit dem Untersuchungsobjekt modifizierte Strahlung kann mittels Strahlungsdetektoren erfasst werden.

Die Abbildungsvorrichtung weist eine Detektoranordnung auf, die zum Erfassen der durch das Untersuchungsobjekt modifizierten Strahlung ausgebildet und vorgesehen ist. Die Detektoranordnung weist mehrere Detektoren auf. Die Detektoren werden auch als Strahlungsdetektoren oder Einzeldetektoren bezeichnet. Die Detektoren können insbesondere als separate Detektoren ausgebildet sein, welche z.B. separat ansteuerbar und auslesbar sein können. Bevorzugt können alle Detektoren der Detektoranordnung gleichartige bzw. identisch ausgebildete Detektoren sein. Jeder der Strahlungsdetektoren ist zum Erfassen der Untersuchungsstrahlung und Erzeugen eines Detektorsignals basierend auf der erfassten Strahlung ausgebildet. Wenn Röntgenstrahlung als Untersuchungsstrahlung verwendet wird, können die Strahlungsdetektoren z.B. Röntgendetektoren zum Erfassen von Röntgenstrahlung sein.

Der zum Aufnehmen des Untersuchungsobjekts vorgesehene Aufnahmeraum der Abbildungsvorrichtung ist im Strahlengang der Untersuchungsstrahlung angeordnet, sodass das Untersuchungsobjekt von der Untersuchungsstrahlung durchstrahlt wird und die Untersuchungsstrahlung von dem Untersuchungsobjekt modifiziert wird. Die Strahlungsdetektoren sind zum Erfassen der von dem Untersuchungsobjekt transmittierten und modifizierten Strahlung vorgesehen und somit im Strahlengang der durch das Untersuchungsobjekt bzw. durch den Aufnahmeraum hindurchgetretenen Strahlung angeordnet.

Die Detektoren der Detektoranordnung können z.B. in Form eines Detektor-Arrays bzw. einer Detektor-Matrix mit mehreren Zeilen und mehreren Spalten angeordnet sein, wobei jede Zeile und jede Spalte mehrere der Detektoren aufweist. Innerhalb der Detektor-Matrix können z.B. alle Detektoren innerhalb einer gemeinsamen Fläche angeordnet sein. Es kann z.B. vorgesehen sein, dass alle Detektoren der Detektoranordnung innerhalb einer ebenen Fläche angeordnet sind, z.B. derart dass die Detektoren eine ebene Detektor-Matrix bilden. Es kann jedoch auch vorgesehen sein, dass alle Detektoren der Detektoranordnung innerhalb einer gekrümmten Fläche angeordnet sind, z.B. innerhalb einer kreisbogenförmig gekrümmten Fläche (d.h. innerhalb einer der Krümmung der Mantelfläche eines Kreiszylinders folgenden Fläche). Bei Ausgestaltung als kreisbogenförmig gekrümmte Detektor-Matrix kann vorgesehen sein, dass die Strahlungsquellposition auf der zugehörigen Krümmungsachse angeordnet ist.

Es kann insbesondere vorgesehen sein, dass die Detektoren der Detektoranordnung in Form einer Detektor-Matrix vorliegen und der Strahlungserzeuger zum Emittieren von divergent von einer punktförmigen Strahlungsquellposition ausgehenden Strahlungspulsen derart ausgebildet ist, dass jeder der Strahlungspulse die gesamte Detektor-Matrix erfasst, d.h. dass die Detektor-Matrix mit allen Strahlungsdetektoren innerhalb des Strahlungskegels der divergenten Strahlungspulse angeordnet ist.

Die Abbildungsvorrichtung weist eine Steuervorrichtung auf, die mit der Detektoranordnung verbunden ist und zum Ansteuern der Detektoranordnung ausgebildet ist. Die Steuervorrichtung kann z.B. zum separaten oder gruppenweisen Ansteuern der Einzeldetektoren der Detektoranordnung ausgebildet sein. Die Steuervorrichtung ist zum Ansteuern der Detektoranordnung derart ausgebildet, dass für jeden der Einzeldetektoren die Strahlungsdetektion alternierend zu vorgegebenen Detektions-Anfangszeitpunkten begonnen und zu vorgegebenen Detektions-Endzeitpunkten beendet wird. Jeder der Einzeldetektoren wird somit alternierend zwischen einem aktiven Zustand, in dem eine Detektion von auf den Einzeldetektor auftreffender Untersuchungsstrahlung erfolgt, und einem passiven Zustand, in dem keine Detektion von auf den Einzeldetektor auftreffender Untersuchungsstrahlung erfolgt, hin und her geschaltet. Zu den Detektions-Anfangszeitpunkten erfolgt ein Übergang vom passiven in den aktiven Zustand, zu den Detektions-Endzeitpunkten erfolgt ein Übergang vom aktiven in den passiven Zustand des jeweiligen Einzeldetektors. Der Zeitraum zwischen einem Detektions-Anfangszeitpunkt und dem darauffolgenden Detektions-Endzeitpunkt eines jeweiligen Einzeldetektors wird auch als Mess-Zeitfenster oder Messfenster bezeichnet. Es kann z.B. vorgesehen sein, dass das Detektorsignal jedes Einzeldetektors über ein jeweiliges Mess-Zeitfenster hinweg integriert und anschließend (vor Beginn des nächsten Mess-Zeitfensters) ausgelesen wird, sodass für jedes Mess-Zeitfenster die Signalintegration erneut beginnt.

Die Steuervorrichtung kann zum Ansteuern jedes der Einzeldetektoren mit vorgegebenen Detektions-Anfangszeitpunkten und vorgegebenen Detektions-Endzeitpunkten ausgebildet sein. Es kann z.B. vorgesehen sein, dass die Steuervorrichtung zum Vorgeben der Detektions-Anfangszeitpunkte und der Detektions-Endzeitpunkte, d.h. zum Bereitstellen der vorgegebenen Detektions-Anfangszeitpunkte und der vorgegebenen Detektions-Endzeitpunkte, ausgebildet ist. Des Weiteren kann die Steuervorrichtung insbesondere zum Ansteuern jedes der Einzeldetektoren mit vorgegebenen Detektions-Anfangszeitpunkten und vorgegebenen Detektions-Endzeitpunkten derart ausgebildet sein, dass der jeweilige Einzeldetektor die vorgegebenen Detektions-Anfangszeitpunkte und die vorgegebenen Detektions-Endzeitpunkte aufweist (d.h. zu den für den jeweiligen Einzeldetektor vorgegebenen Detektions-Anfangszeitpunkten in den aktiven Zustand übergeht und zu den für den jeweiligen Einzeldetektor vorgegebenen Detektions-Endzeitpunkten in den passiven Zustand übergeht).

Die Steuervorrichtung kann somit insbesondere derart ausgebildet sein, dass von ihr für jeden der Einzeldetektoren jeweilige Detektions-Anfangszeitpunkte und Detektions-Endzeitpunkte vorgegeben werden, und dass von ihr die Einzeldetektoren mit den derart vorgegebenen Detektions-Anfangszeitpunkten und Detektions-Endzeitpunkten angesteuert werden, sodass von der Steuervorrichtung jeder der Einzeldetektoren mit den für den jeweiligen Einzeldetektor vorgegebenen Detektions-Anfangszeitpunkten und Detektions-Endzeitpunkten angesteuert wird. Die Steuervorrichtung kann insbesondere zum Ansteuern eines jeweiligen Einzeldetektors mit vorgegebenen Detektions-Anfangszeitpunkten und vorgegebenen Detektions-Endzeitpunkten derart ausgebildet sein, dass für den jeweiligen Einzeldetektor die Strahlungsdetektion zu den vorgegebenen Detektions-Anfangszeitpunkten begonnen und zu den vorgegebenen Detektions-Endzeitpunkten beendet wird. Demgemäß kann also vorgesehen sein, dass von der Steuervorrichtung für jeden Einzeldetektor Detektions-Anfangszeitpunkte und Detektions-Endzeitpunkte vorgegeben werden und die Steuervorrichtung zum Ansteuern der Detektoranordnung derart ausgebildet ist, dass jeder der Einzeldetektoren die für ihn vorgegebenen Detektions-Anfangszeitpunkte und Detektions-Endzeitpunkte aufweist.

Die Steuervorrichtung ist zum Ansteuern der Detektoranordnung derart ausgebildet, dass zumindest einige der Einzeldetektoren unterschiedliche Detektions-Endzeitpunkte aufweisen, d.h. zu unterschiedlichen Zeitpunkten passiv geschaltet werden. Die Steuervorrichtung ist somit zum Ansteuern der Einzeldetektoren der Detektoranordnung derart ausgebildet, dass nicht alle Einzeldetektoren der Detektoranordnung gleichzeitig vom aktiven in den passiven Zustand geschaltet werden.

Die Steuervorrichtung kann insbesondere zum Ansteuern der Detektoranordnung derart ausgebildet sein, dass zumindest einige der Einzeldetektoren mit unterschiedlichen Detektions-Endzeitpunkten angesteuert werden, sodass die Detektions-Endzeitunkte dieser Einzeldetektoren nicht identisch sind. Es kann somit vorgesehen sein, dass (z.B. von der Steuervorrichtung) zumindest für einige der Einzeldetektoren unterschiedliche Detektions-Endzeitpunkte vorgegeben sind bzw. werden, und dass diese Einzeldetektoren mit den vorgegebenen unterschiedlichen Detektions-Endzeitpunkten angesteuert werden. Demgemäß können (z.B. von der Steuervorrichtung) zumindest für einige der Einzeldetektoren unterschiedliche Detektions-Endzeitpunkte vorgegeben sein bzw. werden und diese Einzeldetektoren mit den vorgegebenen unterschiedlichen Detektions-Endzeitpunkten derart angesteuert werden, dass sie diese vorgegebenen unterschiedlichen Detektions-Endzeitpunkte aufweisen (d.h. zu diesen vorgegebenen unterschiedlichen Detektions-Endzeitpunkten vom aktiven in den passiven Zustand wechseln).

Die Abbildungsvorrichtung ist basierend auf den Detektorsignalen der Strahlungsdetektoren der Detektoranordnung zum Erzeugen einer Abbildung des Inneren des durchstrahlten Untersuchungsobjekts ausgebildet, z.B. zum Erzeugen einer tomografischen Abbildung des Inneren des durchstrahlten Untersuchungsobjekts. Wenn Röntgenstrahlung als Untersuchungsstrahlung verwendet wird, kann die Abbildungsvorrichtung z.B. basierend auf den Detektorsignalen zum Erzeugen einer Röntgenabbildung des Untersuchungsobjekts ausgebildet sein. Es kann insbesondere vorgesehen sein, dass die Steuervorrichtung zum Auslesen der Detektoranordnung bzw. der Einzeldetektoren und Erzeugen einer Abbildung des Inneren des durchstrahlten Untersuchungsobjekts basierend auf den so ausgelesenen Detektorsignalen ausgebildet ist.

Diejenigen Einzeldetektoren, welche von der Steuervorrichtung mit unterschiedlichen Detektions-Endzeitpunkten angesteuert werden, weisen unterschiedliche Abstände zu der Strahlungsquellposition auf. Dementsprechend ist vorgesehen, dass die Detektoranordnung zumindest einige (d.h. mindestens zwei) Einzeldetektoren mit unterschiedlichen Abständen zu der Strahlungsquellposition aufweist, welche von der Steuervorrichtung derart angesteuert werden, dass sie zu unterschiedlichen Zeitpunkten vom aktiven in den passiven Zustand geschaltet werden.

Es kann z.B. vorgesehen sein, dass alle Einzeldetektoren, die unterschiedliche Abstände zu der Strahlungsquellposition aufweisen, von der Steuervorrichtung mit unterschiedlichen Detektions-Endzeitpunkten angesteuert werden. Alternativ dazu kann vorgesehen sein, dass mehrere Abstandsintervalle derart vorgegeben sind, dass jeweils solche Einzeldetektoren, deren Abstände zu der Strahlungsquellposition innerhalb desselben Intervalls liegen, von der Steuervorrichtung mit denselben Detektions-Endzeitpunkten angesteuert werden, und solche Einzeldetektoren, deren Abstände zu der Strahlungsquellposition in unterschiedlichen Intervallen liegen, von der Steuervorrichtung mit unterschiedlichen Detektions-Endzeitpunkten angesteuert werden. Die letztgenannte Ausführung ermöglicht z.B. eine Verbesserung der Abbildungsqualität bei reduziertem Aufwand.

Alle Einzeldetektoren, welche den gleichen Abstand zu der Strahlungsquellposition aufweisen, werden von der Steuervorrichtung mit denselben Detektions-Endzeitpunkten angesteuert. Mit anderen Worten werden solche Einzeldetektoren, die gleich weit von der Strahlungsquellposition entfernt sind, von der Steuervorrichtung derart angesteuert, dass sie zu denselben Zeitpunkten vom aktiven in den passiven Zustand geschaltet werden.

Gemäß einer Ausführungsform weisen somit einige oder alle Einzeldetektoren der Detektoranordnung unterschiedliche Abstände zu der Strahlungsquellposition auf, wobei Einzeldetektoren mit unterschiedlichen Abständen zu der Strahlungsquellposition von der Steuervorrichtung mit unterschiedlichen Detektions-Endzeitpunkten angesteuert werden, und wobei Einzeldetektoren mit gleichen Abständen zu der Strahlungsquellposition von der Steuervorrichtung mit gleichen Detektions-Endzeitpunkten angesteuert werden.

Die Strahlungsquellposition ist bevorzugt punktförmig, d.h. bildet bevorzugt eine punktförmige Strahlungsquelle, wobei die Strahlungsquellposition vereinfacht durch einen Punkt gebildet ist und der Abstand zwischen der Strahlungsquellposition und einem jeweiligen Einzeldetektor durch den Abstand zwischen diesem Punkt und dem jeweiligen Einzeldetektor gegeben ist.

Gemäß einer Ausführungsform werden diejenigen Einzeldetektoren, die unterschiedliche Abstände zu der Strahlungsquellposition aufweisen und von der Steuervorrichtung mit unterschiedlichen Detektions-Endzeitpunkten angesteuert werden, derart angesteuert, dass die Detektions-Endzeitpunkte für Einzeldetektoren mit einem größeren Abstand zu der Strahlungsquellposition zeitlich hinter den Detektions-Endzeitpunkten für Einzeldetektoren mit einem demgegenüber kleineren Abstand zu der Strahlungsquellposition liegen. Gemäß dieser Ausführung werden die Einzeldetektoren um so später vom aktiven in den passiven Zustand geschaltet, je größer ihr Abstand zu der Strahlungsquellposition ist.

Gemäß einer Ausführungsform weisen somit einige oder alle Einzeldetektoren der Detektoranordnung unterschiedliche Abstände zu der Strahlungsquellposition auf, wobei Einzeldetektoren mit unterschiedlichen Abständen zu der Strahlungsquellposition von der Steuervorrichtung derart mit unterschiedlichen Detektions-Endzeiten angesteuert werden, dass die Detektions-Endzeitpunkte für Einzeldetektoren mit einem größeren Abstand zu der Strahlungsquellposition zeitlich hinter den Detektions-Endzeitpunkten für Einzeldetektoren mit einem demgegenüber kleineren Abstand zu der Strahlungsquellposition liegen, und wobei Einzeldetektoren mit gleichen Abständen zu der Strahlungsquellposition von der Steuervorrichtung mit gleichen Detektions-Endzeiten angesteuert werden.

Indem nicht für alle Strahlungsdetektoren dieselben Detektions-Endzeitpunkte vorliegen, können z.B. die vorstehend genannten, mit der Verwendung ein und desselben Mess-Zeitfensters für alle Strahlungsdetektoren einhergehenden Nachteile gemindert oder behoben werden und somit eine hohe Abbildungsqualität ermöglicht werden. Insbesondere können die durch unterschiedlich große räumliche Abstände zwischen der Strahlungsquelle und einzelnen Einzeldetektoren bedingten unterschiedlichen Laufzeiten der Pulse von der Quelle bis zum jeweiligen Detektor bei der Erzeugung der Detektorsignale berücksichtigt werden, wodurch z.B. eine einheitliche Berücksichtigung sowohl ungestreuter als auch gestreuter Strahlungsanteile an allen Einzeldetektoren ermöglicht ist. Mittels Verwendung unterschiedlicher Detektions-Endzeitpunkte können insbesondere für unterschiedlich weit von der Strahlungsquelle entfernte Detektoren die Zeiträume zwischen dem Eintreffen der ersten ungestreuten Photonen eines jeweiligen Pulses an dem jeweiligen Einzeldetektor und dem Detektions-Endzeitpunkt des jeweiligen Einzeldetektors für alle Einzeldetektoren gleich groß eingestellt werden. Somit können die Zeitspannen, innerhalb derer überhaupt ungestreute Strahlung an einem jeweiligen Einzeldetektor eintreffen und von demselben detektiert werden kann, für alle Einzeldetektoren gleich groß eingestellt werden, wodurch z.B. eine einheitliche Berücksichtigung ungestreuter Strahlungsanteile an allen Einzeldetektoren ermöglicht ist. Mittels Verwendung unterschiedlicher Detektions-Endzeitpunkte können zudem für unterschiedlich weit von der Strahlungsquelle entfernte Detektoren die Unterschiede in den Streustrahlungsanteilen der jeweiligen Detektorsignale vermindert oder sogar verhindert werden, wodurch die mit derartigen Unterschieden der Streustrahlungsanteile einhergehende Abbildungsverfälschung vermindert oder verhindert werden kann.

Die durch das Untersuchungsobjekt hindurchtretende gepulste Strahlung wird also mittels der Detektoranordnung mit mehreren Einzeldetektoren detektiert, wobei mittels der Einzeldetektoren keine durchgehende Strahlungsdetektion erfolgt, sondern die Einzeldetektoren in zeitlicher Abstimmung auf die Taktung der gepulsten Strahlungsquelle alternierend aktiv und passiv geschaltet werden. Die Ansteuerung der Detektoranordnung erfolgt zeitlich abgestimmt auf die Taktung des Strahlungserzeugers. Dies kann z.B. realisiert werden, indem die Steuervorrichtung sowohl mit der Detektoranordnung als auch mit dem Strahlungserzeuger verbunden ist und zum entsprechend synchronisierten (zeitlich aufeinander abgestimmten) Ansteuern der Detektoranordnung und der Strahlungserzeugungsvorrichtung ausgebildet ist. Demgemäß werden die Detektoranordnung und der Strahlungserzeuger von der Steuervorrichtung derart synchronisiert angesteuert, dass die erläuterten zeitlichen Abfolgen vorliegen.

Der Strahlungserzeuger ist zum Emittieren von Strahlungspulsen ausgehend von einer Strahlungsquellposition ausgebildet, wobei die von der Strahlungsquellposition ausgehende Strahlungsemission alternierend zu vorgegebenen Pulsemissions-Anfangszeitpunkten begonnen und zu vorgegebenen Pulsemissions-Endzeitpunkten beendet wird. Die Zeitspanne zwischen einem Pulsemissions-Anfangszeitpunkt und dem darauffolgenden Pulsemissions-Endzeitpunkt entspricht der Pulsdauer. Die Pulsrate, welche auch als Repetitionsrate oder Pulsfrequenz bezeichnet wird, kennzeichnet die Anzahl der emittierten Pulse pro Zeit. Im Falle einer zeitlich periodischen Pulserzeugung entspricht die Zeitspanne zwischen zwei aufeinanderfolgenden Pulsemissions-Anfangszeitpunkten (oder zwischen zwei aufeinanderfolgenden Pulsemissions-Endzeitpunkten) der Periodendauer der Pulserzeugung. In diesem Fall entspricht die Pulsrate dem Kehrwert der Periodendauer. Der Strahlungserzeuger ist zum Erzeugen von Strahlungspulsen mit einer vorgegebenen Pulsdauer ausgebildet. Der zeitliche Abstand zwischen zwei aufeinanderfolgenden Pulsemissions-Anfangszeitpunkten ist größer als die Pulsdauer und beträgt bevorzugt mindestens das 1,5-fache der Pulsdauer, bevorzugt mindestens das 2-fache der Pulsdauer, wodurch eine Überlagerung von Strahlungsanteilen unterschiedlicher Pulse an den Detektoren verhindert werden kann. Demgemäß liegt ein zeitlicher Abstand zwischen einem Pulsemissions-Endzeitpunkt und dem darauffolgenden Pulsemissions-Anfangszeitpunkt vor, wobei dieser zeitliche Abstand z.B. mindestens die Hälfte der Pulsdauer betragen kann, bevorzugt mindestens genauso groß ist wie die Pulsdauer. Der Strahlungserzeuger ist bevorzugt zum Erzeugen von Pulsen mit einer Pulsdauer von wenigen Nanosekunden (ns) ausgebildet. Es kann z.B. vorgesehen sein, dass die Pulsdauer maximal 100 ns beträgt, bevorzugt maximal 10 ns.

Die Detektoranordnung wird von der Steuervorrichtung derart angesteuert, dass für jeden der Einzeldetektoren die Strahlungsdetektion alternierend zu vorgegebenen Detektions-Anfangszeitpunkten begonnen und zu vorgegebenen Detektions-Endzeitpunkten beendet wird, wobei der Zeitraum zwischen einem Detektions-Anfangszeitpunkt und dem darauffolgenden Detektions-Endzeitpunkt eines jeweiligen Einzeldetektors auch als Mess-Zeitfenster bezeichnet wird.

Die Ansteuerung der Detektoranordnung erfolgt zeitlich abgestimmt auf die Taktung des Strahlungserzeugers derart, dass jedem Strahlungspuls (genau) ein Mess-Zeitfenster jedes Einzeldetektors der Detektoranordnung zugeordnet wird, innerhalb dessen Strahlung des betreffenden Strahlungspulses von dem betreffenden Einzeldetektor detektiert wird. Innerhalb jedes Mess-Zeitfensters wird lediglich Strahlung eines einzigen Strahlungspulses detektiert.

Die Ansteuerung der Detektoranordnung erfolgt insbesondere derart zeitlich abgestimmt auf die Taktung des Strahlungserzeugers, dass einem Strahlungspuls, der zwischen einem vorgegebenen Pulsemissions-Anfangszeitpunkt und einem vorgegeben Pulsemissions-Endzeitpunkt von der Strahlungsquellposition ausgeht, für jeden Einzeldetektor ein Mess-Zeitfenster zugeordnet wird, das zu einem vorgegebenen Detektions-Anfangszeitpunkt beginnt und zu einem vorgegebenen Detektions-Endzeitpunkt endet, wobei der Detektions-Endzeitpunkt zeitlich hinter dem Pulsemissions-Anfangszeitpunkt liegt. Somit wird Strahlung eines Strahlungspulses, der zwischen einem vorgegebenen Pulsemissions-Anfangszeitpunkt und einem vorgegeben Pulsemissions-Endzeitpunkt von der Strahlungsquellposition ausgeht, von jedem Einzeldetektor innerhalb eines Mess-Zeitfensters detektiert, das zu einem vorgegebenen Detektions-Endzeitpunkt endet, der zeitlich hinter dem Pulsemissions-Anfangszeitpunkt des Strahlungspulses (und vor dem Pulsemissions-Anfangszeitpunkt des nachfolgenden Strahlungspulses) liegt.

Somit wird

- die von der Strahlungsquellposition ausgehende Strahlungsemission alternierend zu vorgegebenen Pulsemissions-Anfangszeitpunkten begonnen und zu vorgegebenen Pulsemissions-Endzeitpunkten beendet, und
- von jedem der Einzeldetektoren die Strahlungsdetektion alternierend zu vorgegebenen Detektions-Anfangszeitpunkten begonnen und zu vorgegebenen Detektions-Endzeitpunkten beendet,
- wobei jeweils ein (einziger) Detektions-Endzeitpunkt zeitlich zwischen zwei aufeinanderfolgenden Pulsemissions-Anfangszeitpunkten liegt, und jeweils ein (einziger) Detektions-Anfangszeitpunkt zeitlich zwischen zwei aufeinanderfolgenden Detektions-Endzeitpunkten liegt (wobei jedoch der Detektions-Endzeitpunkt und der Detektions-Anfangszeitpunkt, die zwischen zwei aufeinanderfolgenden Detektions-Endzeitpunkten liegen, nicht zu demselben Mess-Zeitfenster gehören müssen).

Die Steuervorrichtung ist also zum Ansteuern der Detektoranordnung derart ausgebildet, dass für jeden Einzeldetektor jeweils ein (einziger) Detektions-Endzeitpunkt zeitlich zwischen zwei aufeinanderfolgenden Pulsemissions-Anfangszeitpunkten angeordnet ist. Die Steuervorrichtung ist insbesondere zum Ansteuern der Detektoranordnung derart ausgebildet, dass für jeden Einzeldetektor das einem jeweiligen Strahlungspuls zugeordnete Mess-Zeitfenster zeitlich nach dem Pulsemissions-Anfangszeitpunkt des betreffenden Pulses endet. Die zugehörigen Detektions-Anfangszeitpunkte liegen zeitlich vor den Detektions-Endzeitpunkten, wobei unterschiedliche Ausgestaltungen möglich sind.

Die Detektoren der Detektoranordnung sind bevorzugt schnell schaltbare Detektoren, welche z.B. Schaltzeiten im Nanosekunden-Bereich ermöglichen, sodass ein schnelles Schalten der Detektoren ermöglicht ist, was z.B. zu einem effektiven Betrieb mit kurzen Pulsdauern und einer effektiven Streustrahlungsunterdrückung beitragen kann. Die Detektoren sind bevorzugt Halbleiterdetektoren, d.h. Detektoren bei denen darauf auftreffende Strahlung freie Ladungsträger in einem Halbleitermaterial erzeugt, welche z.B. an Elektroden in Form eines Fotostroms abgenommen und in ein Spannungssignal gewandelt werden können. Es kann z.B. vorgesehen sein, dass das Detektorsignal während des Mess-Zeitfensters integriert und nach dem Detektions-Endzeitpunkt an eine Speicherelektronik transferiert wird. Die Detektoren können auch als indirekte Wandler ausgeführt sein, von denen die Strahlung mittels eines Szintillationskristalls in sichtbares Licht umgewandelt wird, das dann z.B. von einer Fotodiode gemessen wird.

Gemäß einer Ausführungsform ist die Steuervorrichtung zum Ansteuern der Detektoranordnung derart ausgebildet, dass für jeden Einzeldetektor das einem jeweiligen Strahlungspuls zugeordnete Mess-Zeitfenster vor dem Pulsemissions-Anfangszeitpunkt des betreffenden Pulses beginnt. Gemäß dieser Ausführung ist die Steuervorrichtung zum Ansteuern der Detektoranordnung derart ausgebildet, dass für jeden Einzeldetektor der Detektions-Anfangszeitpunkt eines Mess-Zeitfensters, das zeitlich nach einem unmittelbar vorhergehenden Pulsemissions-Anfangszeitpunkt endet, zeitlich vor diesem Pulsemissions-Anfangszeitpunkt liegt.

Somit ist für jeden Einzeldetektor zwischen zwei aufeinanderfolgenden Pulsemissions-Anfangszeitpunkten jeweils ein (einziger) Detektions-Anfangszeitpunkt und ein (einziger) Detektions-Endzeitpunkt derart angeordnet, dass der Detektions-Anfangszeitpunkt zeitlich hinter dem Detektions-Endzeitpunkt liegt (wobei der Detektions-Endzeitpunkt und der Detektions-Anfangszeitpunkt zu unterschiedlichen Mess-Zeitfenstern gehören). Demgemäß liegt jeder Pulsemissions-Anfangszeitpunkt innerhalb des Mess-Zeitfensters, das zur Detektion des von der Strahlungsquellposition aus beginnend zu dem Pulsemissions-Anfangszeitpunkt abgestrahlten Pulses dient. Gemäß der vorliegenden Ausführungsform werden somit zunächst die Einzeldetektoren in den aktiven Zustand versetzt, danach beginnt die Pulsemission an der Strahlungsquellposition, und danach werden die Einzeldetektoren wieder in den passiven Zustand versetzt. Indem die Einzeldetektoren jeweils vor Beginn der Pulsemission in den aktiven Zustand versetzt werden, verbleibt für die Einzeldetektoren bis zum Eintreffen der ersten Strahlungsanteile eine Einstellzeit zum Erreichen eines stabilen Betriebszustandes bzw. einer stabilen Detektionscharakteristik, wodurch eine hohe Abbildungsqualität zusätzlich unterstützt werden kann.

Die Detektoren werden derart angesteuert, dass zumindest einige der Detektoren unterschiedliche Detektions-Endzeitpunkte aufweisen, d.h. zu unterschiedlichen Zeitpunkten vom aktiven in den passiven Zustand geschaltet werden. Bezüglich der Detektions-Anfangszeitpunkte kann z.B. vorgesehen sein, dass zumindest die Detektoren mit unterschiedlichen Detektions-Endzeitpunkten von der Steuervorrichtung auch mit unterschiedliche Detektions-Anfangszeitpunkten angesteuert werden, d.h. zu unterschiedlichen Zeitpunkten vom passiven in den aktiven Zustand geschaltet werden. Es kann jedoch z.B. auch vorgesehen sein, dass alle Detektoren der Detektoranordnung mit gleichen Detektions-Anfangszeitpunkten angesteuert werden, d.h. zu gleichen Zeitpunkten vom passiven in den aktiven Zustand geschaltet werden.

Gemäß einer Ausführungsform ist demgemäß die Steuervorrichtung zum Ansteuern der Detektoranordnung derart ausgebildet, dass alle Einzeldetektoren dieselben Detektions-Anfangszeitpunkte aufweisen, d.h. dass alle Einzeldetektoren jeweils gleichzeitig vom passiven in den aktiven Zustand geschaltet werden (unabhängig davon ob sie mit gleichen oder unterschiedlichen Detektions-Endzeitpunkten angesteuert werden). Es kann insbesondere vorgesehen sein, dass alle Einzeldetektoren derart mit denselben Detektions-Anfangszeiten angesteuert werden, dass für alle Einzeldetektoren die einem jeweiligen Strahlungspuls zugeordneten Mess-Zeitfenster vor dem Pulsemissions-Anfangszeitpunkt des betreffenden Pulses beginnen. Indem alle Einzeldetektoren unabhängig von ihren Detektions-Endzeitpunkten jeweils gleichzeitig vom passiven in den aktiven Zustand geschaltet werden, kann die Ansteuerung der Detektoranordnung vereinfacht werden.

Für Detektoren mit unterschiedlichen Abständen von der Strahlungsquellposition benötigen die Pulse bzw. die Photonen der Pulse unterschiedliche Laufzeiten von der Strahlungsquellposition bis zu dem jeweiligen Detektor. Dadurch kann die Zeitspanne zwischen dem Eintreffen der ersten Strahlungsanteile eines jeweiligen Pulses und dem Detektions-Endzeitpunkt des zugehörigen Mess-Zeitfensters für unterschiedliche Detektoren unterschiedlich groß sein. Dadurch kann es zu Bildverfälschungen kommen, da diese Zeitspanne den Zeitbereich darstellt, innerhalb dessen überhaupt Strahlung an dem Detektor eintreffen und detektiert werden kann. Indem von der Steuervorrichtung Einzeldetektoren mit unterschiedlich großen Abständen zu der Strahlungsquellposition mit unterschiedlichen Detektions-Endzeitpunkten angesteuert werden können, kann bei der Detektion berücksichtigt werden, dass die Pulse bzw. Photonen für unterschiedliche Abstände zwischen der Strahlungsquelle und einem jeweiligen Einzeldetektor auch unterschiedliche Laufzeiten zum Zurücklegen der unterschiedlichen Abstände benötigen, und/oder dass ungestreute und gestreute Photonen unterschiedliche Laufzeiten aufweisen.

Gemäß einer Ausführungsform werden die Einzeldetektoren insbesondere derart angesteuert, dass die Zeitspanne zwischen dem Eintreffen der ersten Strahlungsanteile eines jeweiligen Pulses und dem Detektions-Endzeitpunkt des dem Puls zugeordneten Mess-Zeitfensters für alle Einzeldetektoren gleich groß ist. Dadurch können die Zeitspannen, innerhalb derer überhaupt ungestreute Strahlung an einem jeweiligen Einzeldetektor eintreffen und von demselben detektiert werden kann, für alle Einzeldetektoren gleich groß gehalten werden, wodurch z.B. eine einheitliche Berücksichtigung ungestreuter Strahlungsanteile an allen Einzeldetektoren ermöglicht ist. Zudem können für unterschiedlich weit von der Strahlungsquelle entfernte Detektoren die Unterschiede in den Streustrahlungsanteilen der jeweiligen Detektorsignale vermindert oder sogar verhindert werden, wodurch die mit derartigen Unterschieden der Streustrahlungsanteile einhergehende Abbildungsverfälschung vermindert oder verhindert werden kann.

Die Zeit, welche Untersuchungsstrahlung von der Strahlungsquellposition aus bis zum Erreichen eines jeweiligen Einzeldetektors der Detektoranordnung benötigt, wird auch als Laufzeit oder Strahlungs-Laufzeit bezeichnet. Die Strahlungs-Laufzeit entspricht z.B. der Zeit, welche ein Strahlungsanteil (z.B. ein Photon) der Untersuchungsstrahlung ausgehend von der Strahlungsquellposition bis zum Erreichen eines jeweiligen Einzeldetektors benötigt. Ungestreute Strahlungsanteile, welche das Untersuchungsobjekt ohne Streuung durchlaufen und somit auf kürzestem Weg direkt (z.B. geradlinig) von der Strahlungsquellposition zu dem jeweiligen Einzeldetektor gelangen, weisen kürzere Strahlungs-Laufzeiten auf als gestreute Strahlungsanteile, welche im Untersuchungsobjekt aufgrund von Streuung (z.B. Compton-Streuung) Richtungsänderungen erfahren und somit nicht auf dem kürzesten Weg von der Strahlungsquellposition zu dem jeweiligen Einzeldetektor gelangen. Die Strahlungs-Laufzeit ungestreuter Strahlungsanteile wird auch als Direkt-Laufzeit bezeichnet. Für gestreute Strahlungsanteile kann der bis zum Erreichen des jeweiligen Einzeldetektors zurückgelegte Weg und somit auch die Strahlungs-Laufzeit wesentlich größer sein als für ungestreute Strahlungsanteile, z.B. wenn eine starke Streuung mit großen Streuwinkeln und/oder einer Vielzahl von Streuprozessen vorliegt. Je mehr Streuung die Strahlungsanteile erfahren, desto mehr vergrößert sich die Laufzeit dieser gestreuten Strahlungsanteile gegenüber der Laufzeit ungestreuter Strahlungsanteile. Je mehr Streuung die Strahlungsanteile erfahren, desto größer wird zudem die durch sie verursachte Bildverfälschung.

Die Direkt-Laufzeit, welche ungestreute Strahlungsanteile ausgehend von der Strahlungsquellposition bis zum Erreichen eines jeweiligen Einzeldetektors der Detektoranordnung benötigen, entspricht der Zeit, welche zum Zurücklegen des Abstandes zwischen der Strahlungsquellposition und dem jeweiligen Einzeldetektor mit Lichtgeschwindigkeit benötigt wird. Die Direkt-Laufzeit entspricht somit dem Quotienten aus dem Abstand des jeweiligen Einzeldetektors von der Strahlungsquellposition und der Lichtgeschwindigkeit. Zur Ermittlung der Direkt-Laufzeit kann die im Vakuum vorliegende Lichtgeschwindigkeit verwendet werden, da dies für die vorliegenden Anwendungen und die dafür geeigneten Photonenenergien hinreichend genau ist.

Gemäß der vorstehend erläutertem Ausführungsform werden die Einzeldetektoren derart angesteuert, dass die Zeitspanne zwischen dem Eintreffen der ersten Strahlungsanteile eines jeweiligen Pulses und dem Detektions-Endzeitpunkt des dem Puls zugeordneten Mess-Zeitfensters für alle Einzeldetektoren gleich groß ist. Die Zeitspanne zwischen dem Eintreffen der ersten Strahlungsanteile eines Pulses an einem Einzeldetektor und dem Detektions-Endzeitpunkt des dem Puls zugeordneten Mess-Zeitfensters dieses Einzeldetektors ergibt sich aus dem zeitlichen Abstand zwischen dem Pulsemissions-Anfangszeitpunkt des Pulses und dem (darauf folgenden) Detektions-Endzeitpunkt, verringert um die Direkt-Laufzeit, welche ungestreute Strahlungsanteile ausgehend von der Strahlungsquellposition bis zum Erreichen des Einzeldetektors benötigen.

Somit ist gemäß dieser Ausführungsform die Steuervorrichtung zum Ansteuern der Detektoranordnung derart ausgebildet, dass für jeden Einzeldetektor der zeitliche Abstand zwischen einem Pulsemissions-Anfangszeitpunkt und dem darauf folgenden Detektions-Endzeitpunkt, verringert um die Direkt-Laufzeit, welche ungestreute Strahlungsanteile ausgehend von der Strahlungsquellposition bis zum Erreichen des Einzeldetektors benötigen, gleich groß ist. Mit anderen Worten ausgedrückt ist gemäß dieser Ausführungsform die Steuervorrichtung zum Ansteuern der Detektoranordnung derart ausgebildet, dass die Differenz zwischen dem zeitlichen Abstand, der zwischen einem Pulsemissions-Anfangszeitpunkt und dem darauf folgenden Detektions-Endzeitpunkt eines jeweiligen Einzeldetektors vorliegt, und der Direkt-Laufzeit, welche ungestreute Strahlungsanteile ausgehend von der Strahlungsquellposition bis zum Erreichen des jeweiligen Einzeldetektors benötigen, für alle Einzeldetektoren gleich groß ist. Die Direkt-Laufzeit wiederum entspricht der Zeit, welche zum Überbrücken des Abstandes zwischen der Strahlungsquellposition und dem jeweiligen Einzeldetektor mit Lichtgeschwindigkeit benötigt wird, wobei die im Vakuum vorliegende Lichtgeschwindigkeit herangezogen werden kann. Die Direkt-Laufzeit entspricht somit dem Quotienten aus dem Abstand des jeweiligen Einzeldetektors von der Strahlungsquellposition und der Lichtgeschwindigkeit.

Somit kann gemäß der vorliegenden Ausführungsform die Steuervorrichtung zum Ansteuern der Detektoranordnung derart ausgebildet sein, dass für alle Einzeldetektoren der zeitliche Abstand zwischen einem jeweiligen Pulsemissions-Anfangszeitpunkt und dem darauf folgenden Detektions-Endzeitpunkt, verringert um den Quotienten aus dem Abstand des jeweiligen Einzeldetektors von der Strahlungsquellposition und der Lichtgeschwindigkeit, gleich groß ist.

Die erläuterten Ansteuerungen der Detektoranordnung in zeitlicher Abstimmung auf die Strahlungserzeugungsvorrichtung können z.B. realisiert werden, indem die Steuervorrichtung sowohl mit der Detektoranordnung als auch mit der Strahlungserzeugungsvorrichtung verbunden ist und zum entsprechend synchronisierten (zeitlich aufeinander abgestimmten) Ansteuern der Detektoranordnung und der Strahlungserzeugungsvorrichtung ausgebildet ist.

Mittels der vorstehend erläuterten Ausführungsformen kann ein hochqualitatives Abbilden des Inneren eines Untersuchungsobjekts unterstützt werden. Zudem kann mittels bestimmter Ausführungsformen die durch Streustrahlungsanteile der Detektorsignale verursachte Abbildungsverschlechterung und -fälschung effektiv weiter vermindert werden.

Bei der Transmissionsbildgebung mittels Durchleuchtens eines Untersuchungsobjekts mit elektromagnetischer Strahlung bzw. Photonen tritt Compton-Streuung auf, wobei ein Teil der Photonen der zur Bildgebung genutzten elektromagnetischen Strahlung unter Richtungsänderung ein- oder mehrmals an Teilchen (insbesondere Elektronen) des Materials des Untersuchungsobjektes gestreut wird. Ein Teil der derart gestreuten Photonen trifft auf die zur Strahlungsdetektion verwendeten Strahlungsdetektoren und trägt somit zum Detektorsignal bei bzw. bildet einen Teil des Detektorsignals, wobei dieser auf die gestreuten Photonen zurückgehende Anteil des Detektorsignals auch als Streustrahlungsanteil des Detektorsignals bezeichnet wird. Die Detektion derartiger gestreuter Photonen führt zu einer Bildverfälschung, z.B. da bei der Transmissionsbildgebung das Vorliegen der jeweils eingestellten Durchstrahlungsgeometrie zugrundegelegt wird, die gestreuten Photonen jedoch aufgrund der Richtungsänderung von dieser zugrundegelegten Durchstrahlungsgeometrie abweichen. Der Streustrahlungsanteil kann sich z.B. als ein Gleichanteil bzw. Grundgrauwert in dem erzeugten Bild äußern. Dadurch werden die Kontrastauflösung herabgesetzt und das Grundrauschniveau erhöht, was z.B. zu einer Reduktion der Detailerkennbarkeit im Bild führt. Den mittels gestreuter Photonen hervorgerufenen Bildverfälschungen kann mittels unterschiedlicher Maßnahmen begegnet werden.

So kann z.B. eine elektronische Energiediskriminierung zur Unterscheidung von gestreuten und ungestreuten Photonen sowie zur Selektion und Detektion ungestreuter Photonen verwendet werden, da die Compton-Streuung mit einer Verringerung der Energie des gestreuten Photons einhergeht. Eine derartige Energiediskriminierung geht jedoch mit einem apparativen Mehraufwand einher und ist nur möglich, wenn die verwendete Strahlungsquelle im Wesentlichen monoenergetische Strahlung bereitstellt und somit die Energie ungestreuter Photonen bekannt und auf einen eng begrenzten Energiebereich eingeschränkt ist. Dies ist jedoch nicht immer der Fall, so weist z.B. bei der Röntgenbildgebung mittels Bremsstrahlung die Strahlung (auch ohne Compton-Streuung) ein breites Energiespektrum auf, wobei alle Photonen des Spektrums zum Messsignal beitragen können, z.B. indem das Detektorsignal aus dem Produkt der Photonenanzahl und der Photonenenergie gebildet wird.

Als ein anderes Beispiel kann der Streustrahlungsanteil im Bild mittels eines Kollimators reduziert werden, mittels dessen die auf den Detektor treffende Strahlung richtungskollimiert wird, sodass Strahlungsanteile mit einer zu großen Richtungsabweichung gegenüber ungestreuter Strahlung mittels des Kollimators ausgeblendet werden und gar nicht erst auf dem Detektor auftreffen. Eine derartige Kollimation geht jedoch mit einem apparativen Mehraufwand einher und erfordert zusätzlichen Bauraum im bildgebenden System, wobei das Erreichen eines hinreichenden Schachtverhältnisses für eine wirkungsvolle Kollimation teils einen großen Bauraum erfordert. Zudem bestehen entsprechende Kollimatoren aus Material hoher Ordnungszahl und sind daher schwer. Zudem ist eine Kollimation in bestimmten Fällen aufgrund der apparativen oder geometrischen Gegebenheiten nur begrenzt oder gar nicht möglich.

Als ein weiteres Beispiel kann der Streustrahlungsanteil im Bild mittels einer Strahlungsfeldsimulation näherungsweise abgeschätzt und herausgerechnet werden. Dies erfordert jedoch zusätzlichen Rechenaufwand und setzt voraus, dass die Geometrien und das Absorptionsverhalten des Untersuchungsobjektes bekannt sind, sodass diese Möglichkeit oft an der Unbekanntheit des Untersuchungsobjektes scheitert.

Ausführungsformen der Erfindung ermöglichen eine effektive Reduzierung der Streustrahlungsanteile der Detektorsignale und dadurch eine weitere Verbesserung der Abbildungsqualität (insbesondere des Abbildungskontrastes), ohne hierfür die dafür herkömmlich verwendeten Methoden wie Energiediskriminierung, Kollimation und/oder Strahlungsfeldsimulation zu erfordern (wobei die genannten Maßnahmen jedoch nichtsdestotrotz zusätzlich eingesetzt werden können).

Gemäß einer Ausführungsform ist die Steuervorrichtung zum Ansteuern der Detektoranordnung derart ausgebildet, dass für jeden Einzeldetektor der zeitliche Abstand zwischen einem Pulsemissions-Anfangszeitpunkt und dem darauffolgenden Detektions-Endzeitpunkt kleiner ist als die Summe aus der Pulsdauer und der Direkt-Laufzeit, welche ungestreute Strahlungsanteile ausgehend von der Strahlungsquellposition bis zum Erreichen des jeweiligen Einzeldetektors benötigen. Der auf einen Pulsemissions-Anfangszeitpunkt folgende Detektions-Endzeitpunkt ist der Detektions-Endzeitpunkt des Mess-Zeitfensters, das zur Detektion des von der Strahlungsquellposition aus beginnend zu dem Pulsemissions-Anfangszeitpunkt abgestrahlten Pulses dient und somit diesem Puls zugeordnet ist. Für einen Puls, der zu einem vorgegebenen Pulsemissions-Anfangszeitpunkt beginnend und mit einer vorgegebenen Pulsdauer von der Strahlungsquellposition ausgeht, erreichen die letzten ungestreuten Strahlungsanteile des Pulses einen jeweiligen Einzeldetektor zu einem Zeitpunkt, der in einem der Summe aus der Pulsdauer und der Direkt-Laufzeit entsprechenden zeitlichen Abstand hinter dem Pulsemissions-Anfangszeitpunkt liegt. Gestreute Strahlungsanteile weisen längere Laufzeiten auf als ungestreute Strahlungsanteile, sodass nach diesem Zeitpunkt zwar keine ungestreuten Strahlungsanteile mehr an dem Einzeldetektor eintreffen, jedoch nach diesem Zeitpunkt noch gestreute Strahlungsanteile an dem Einzeldetektor eintreffen. Indem die Strahlungsdetektion mittels der Einzeldetektoren jeweils vor diesem Zeitpunkt beendet wird, werden nach diesem Zeitpunkt an einem jeweiligen Einzeldetektor eintreffende gestreute Strahlungsanteile nicht mehr detektiert, sodass der Streustrahlungsanteil des zugehörigen Detektorsignals reduziert werden kann. Da der Streustrahlungsanteil sich z.B. in einem Gleichanteil bzw. Grundgrauwert und einer verringerten Kontrastauflösung in der erzeugten Abbildung äußert, kann mittels eines reduzierten Streustrahlungsanteils z.B. die Detailerkennbarkeit in der erzeugten Abbildung verbessert werden.

Gemäß einer weiteren Ausführungsform ist die Steuervorrichtung zum Ansteuern der Detektoranordnung derart ausgebildet, dass für jeden Einzeldetektor der zeitliche Abstand zwischen einem Pulsemissions-Anfangszeitpunkt und dem darauffolgenden Detektions-Endzeitpunkt kleiner ist als das Doppelte der Direkt-Laufzeit, welche ungestreute Strahlungsanteile ausgehend von der Strahlungsquellposition bis zum Erreichen des jeweiligen Einzeldetektors benötigen. Es kann z.B. vorgesehen sein, dass für jeden Einzeldetektor der zeitliche Abstand zwischen einem Pulsemissions-Anfangszeitpunkt und dem darauffolgenden Detektions-Endzeitpunkt kleiner ist als das 1,5-fache der jeweils zugehörigen Direkt-Laufzeit, z.B. kleiner als das 1,2-fache der jeweils zugehörigen Direkt-Laufzeit.

Indem für jeden Einzeldetektor der zeitliche Abstand zwischen einem jeweiligen Pulsemissions-Anfangszeitpunkt und dem (unmittelbar) nachfolgenden Detektions-Endzeitpunkt maximal dem Doppelten (d.h. dem 2-fachen) der Direkt-Laufzeit entspricht, welche ungestreute Photonen des jeweiligen Strahlungspulses von der Strahlungsquellposition bis zum Erreichen des jeweiligen Einzeldetektors benötigen, kann die Detektion gestreuter Photonen effektiv unterdrückt und auf entsprechend schwach gestreute Photonen beschränkt werden, wodurch der Beitrag stark gestreuter Photonen zur Bilderzeugung und somit auch die Bildverfälschung reduziert werden kann. Durch die derart ermöglichte Unterdrückung stark gestreuter Photonen können die Streustrahlungsanteile der Detektorsignale gering gehalten werden und somit die Detektorsignale weitgehend frei von Streustrahlungsanteilen gehalten werden, wodurch eine Verbesserung der Bildqualität, insbesondere eine Steigerung der Kontrastauflösung und der Detailerkennbarkeit in den erzeugten Bildern, ermöglicht ist.

Gestreute Strahlung legt bis zum Erreichen eines jeweiligen Einzeldetektors einen längeren Weg zurück als ungestreute Strahlung. Bei Beschränkung des zeitlichen Abstandes zwischen einem Pulsemissions-Anfangszeitpunkt und dem darauf folgenden Detektions-Endzeitpunkt auf maximal das Doppelte der Direkt-Laufzeit, welche ungestreute Strahlungsanteile ausgehend von der Strahlungsquellposition bis zum Erreichen des jeweiligen Einzeldetektors benötigen, wird die Detektion gestreuter Streustrahlungsanteile auf solche Streustrahlungsanteile begrenzt, für welche der bis zum Erreichen eines jeweiligen Einzeldetektors zurückgelegte Weg maximal dem Doppelten des Abstandes zwischen der Strahlungsquellposition und dem jeweiligen Einzeldetektor beträgt. Da für die zur Transmissionsbildgebung verwendeten Photonenenergien die Compton-Streuung ein Streumaximum bei ca. 60° aufweist, ermöglicht eine Beschränkung des zeitlichen Abstandes zwischen einem Pulsemissions-Anfangszeitpunkt und dem darauf folgenden Detektions-Endzeitpunkt auf maximal das Doppelte der Direkt-Laufzeit eine effektive Reduzierung des Streustrahlungsanteils der Detektorsignale.

Gemäß einer weiteren Ausführungsform ist zudem der zeitliche Abstand zwischen einem Pulsemissions-Endzeitpunkt und dem darauffolgenden Detektions-Anfangszeitpunkt für jeden Einzeldetektor größer als die Direkt-Laufzeit, welche ungestreute Strahlungsanteile von der Strahlungsquellposition bis zum Erreichen des jeweiligen Einzeldetektors benötigen, und beträgt z.B. mindestens das 2-fache der Direkt-Laufzeit, bevorzugt mindestens das 5-fache der Direkt-Laufzeit.

Für unterschiedliche Einzeldetektoren können unterschiedliche Direkt-Laufzeiten vorliegen, wobei die größte dieser Direkt-Laufzeiten auch als Maximal-Direktlaufzeit bezeichnet wird. Die Maximal-Direktlaufzeit bezeichnet also die Direkt-Laufzeit für den größten zwischen einem Einzeldetektor und der Strahlungsquellposition vorliegenden Abstand. Die Maximal-Direktlaufzeit bezeichnet somit die Zeit, welche zum Zurücklegen des größten zwischen einem Einzeldetektor und der Strahlungsquellposition vorliegenden Abstandes mit Lichtgeschwindigkeit benötigt wird.

Gemäß der vorliegenden Ausführungsform kann insbesondere vorgesehen sein, dass der zeitliche Abstand zwischen einem Pulsemissions-Endzeitpunkt und dem darauffolgenden Detektions-Anfangszeitpunkt für jeden Einzeldetektor größer ist als die Maximal-Direktlaufzeit, und z.B. mindestens das 2-fache der Maximal-Direktlaufzeit beträgt, bevorzugt mindestens das 5-fache der Maximal-Direktlaufzeit.

Alternativ oder zusätzlich dazu kann vorgesehen sein, dass der zeitliche Abstand zwischen einem Pulsemissions-Endzeitpunkt und dem darauffolgenden Detektions-Anfangszeitpunkt für jeden Einzeldetektor größer ist als die Pulsdauer der Strahlungspulse, und z.B. mindestens das 2-fache der Pulsdauer beträgt, bevorzugt mindestens das 5-fache der Pulsdauer, wobei die Pulsdauer z.B. größer sein kann als die Maximal-Direktlaufzeit.

Indem der zeitliche Abstand zwischen einem Pulsemissions-Endzeitpunkt und dem Beginn des nachfolgenden Mess-Zeitfensters (mittels einer entsprechenden Ansteuerung der Detektoranordnung und ggf. des Strahlungserzeugers) entsprechend groß eingestellt wird, kann eine Verfälschung der einem Strahlungspuls zugeordneten, in einem Mess-Zeitfenster generierten Detektorsignale durch Streustrahlung des vorhergehenden Strahlungspulses vermindert oder verhindert werden.

Gemäß einer Ausführungsform ist die Strahlungserzeugungsvorrichtung zum Erzeugen von Pulsen elektromagnetischer Strahlung ausgebildet, die Photonen mit Photonenenergien bzw. Quantenenergien von mindestens 80 keV aufweisen oder daraus bestehen. Bei der Transmissionsbildgebung in diesem Energiebereich ist eine besonders effektive Steigerung der Abbildungsqualität ermöglicht, insbesondere mittels Reduzierung bzw. Unterdrückung der in diesem Energiebereich wesentlichen, durch Compton-Streuung generierten Streustrahlungsanteile. Bei derart hohen Photonenenergien ist die Lichtgeschwindigkeit im Wesentlichen unabhängig vom Ausbreitungsmedium und entspricht im Wesentlichen der Vakuum-Lichtgeschwindigkeit, sodass insbesondere bei der Ermittlung der Direkt-Laufzeit die Vakuum-Lichtgeschwindigkeit herangezogen werden kann. Es kann z.B. vorgesehen sein, dass die Strahlungspulse Photonen mit einer Quantenenergie von mindestens 100 keV aufweisen oder daraus bestehen, insbesondere Photonen mit einer Quantenenergie von mindestens 120 keV (wobei keV für Kilo-Elektronenvolt steht). Demgemäß kann z.B. vorgesehen sein, dass die Untersuchungsstrahlung Röntgenstrahlung (mit Quantenenergien bis 120 keV) oder Gammastrahlung (mit Quantenenergien von mindestens 120 keV) aufweist oder daraus besteht. Die Strahlungserzeugungsvorrichtung kann z.B. zum Erzeugen monoenergetischer Strahlungspulse ausgebildet sein, wobei jeder Strahlungspuls aus Photonen mit einer vorgegebenen Quantenenergie besteht. Es kann jedoch auch vorgesehen sein, dass die Strahlungserzeugungsvorrichtung zum Erzeugen multienergetischer Strahlungspulse ausgebildet ist, wobei jeder Strahlungspuls aus Photonen mehrerer unterschiedlicher Quantenenergien besteht.

Gemäß einem weiteren Aspekt der Erfindung wird ein Verfahren zur Transmissionsbildgebung zum Abbilden des Inneren eines Untersuchungsobjekts bereitgestellt, wobei das Verfahren auch als Abbildungsverfahren bezeichnet wird. Bei dem Abbildungsverfahren kann es sich z.B. um ein tomographisches Abbildungsverfahren handeln. Die vorstehend mit Bezug auf die Abbildungsvorrichtung beschriebenen Merkmale gelten analog für das Abbildungsverfahren, sodass im Folgenden lediglich knapp auf die entsprechenden Ausgestaltungen des Abbildungsverfahrens eingegangen wird und im Übrigen hiermit auf die entsprechenden Erläuterungen hinsichtlich der Abbildungsvorrichtung verwiesen wird.

Gemäß dem Abbildungsverfahren wird das Untersuchungsobjekt mit von einer Strahlungsquellposition ausgehenden Pulsen elektromagnetischer Strahlung durchstrahlt. Die durch das Untersuchungsobjekt hindurchtretende Strahlung wird mittels einer Detektoranordnung mit mehreren Detektoren detektiert, wobei die Detektoranordnung derart angesteuert wird, dass für jeden der Detektoren die Strahlungsdetektion alternierend zu vorgegebenen Detektions-Anfangszeitpunkten begonnen und zu vorgegebenen Detektions-Endzeitpunkten beendet wird. Die Detektoranordnung wird zudem derart angesteuert, dass zumindest einige der Detektoren unterschiedliche Detektions-Endzeitpunkte aufweisen, wobei die Detektoren, welche unterschiedliche Detektions-Endzeitpunkte aufweisen, unterschiedliche Abstände zu der Strahlungsquellposition aufweisen. Die Ansteuerung der Detektoranordnung erfolgt z.B. mittels einer entsprechend ausgebildeten Steuervorrichtung, welche zudem auch mit dem zum Erzeugen der Pulse verwendeten Strahlungserzeuger verbunden sein kann und zum Ansteuern des Strahlungserzeugers ausgebildet sein kann.

Ausführungsformen dieses Verfahrens - insbesondere hinsichtlich der Abhängigkeit der Detektions-Endzeitpunkte unterschiedlicher Einzeldetektoren von deren jeweiligem Abstand zur Strahlungsquellposition (bzw. des Zusammenhangs der Detektions-Endzeitpunkte unterschiedlicher Einzeldetektoren mit deren jeweiligem Abstand zur Strahlungsquellposition), der zeitlichen Abfolge der Detektions-Anfangszeitpunkte und der Detektions-Endzeitpunkte der Einzeldetektoren sowie der Pulsemissions-Anfangszeitpunkte und der Pulsemissions-Endzeitpunkte, der Ansteuerung der Detektoranordnung in zeitlicher Abstimmung auf die Strahlungspulse und der Realisierungsmöglichkeiten einer derartigen Ansteuerung, und der verwendeten Untersuchungsstrahlung - ergeben sich aus der entsprechenden Beschreibung der Abbildungsvorrichtung.

Die Erfindung wird nachfolgend anhand von Ausführungsbeispielen mit Bezugnahme auf die beiliegenden Figuren erläutert, wobei gleiche oder ähnliche Merkmale mit gleichen Bezugszeichen versehen sind, hierbei zeigen schematisch:

**Figur 1**      eine Abbildungsvorrichtung gemäß einer Ausführungsform beim Durchstrahlen eines Untersuchungsobjekts,

**Figur 1A**     eine Abbildungsvorrichtung gemäß einer weiteren Ausführungsform beim Durchstrahlen eines Untersuchungsobjekts,

**Figur 2**      einen Zeitstrahl zur Veranschaulichung der Zeitabfolge bezüglich zweier Einzeldetektoren mit unterschiedlichen Abständen zu der Strahlungsquellposition,

**Figur 3**      einen Zeitstrahl zur Veranschaulichung der Zeitabfolge bezüglich eines jeweiligen Einzeldetektors, und

**Figur 4**      eine seitliche Schnittdarstellung zur Veranschaulichung von ungestreuter und gestreuter Strahlung.

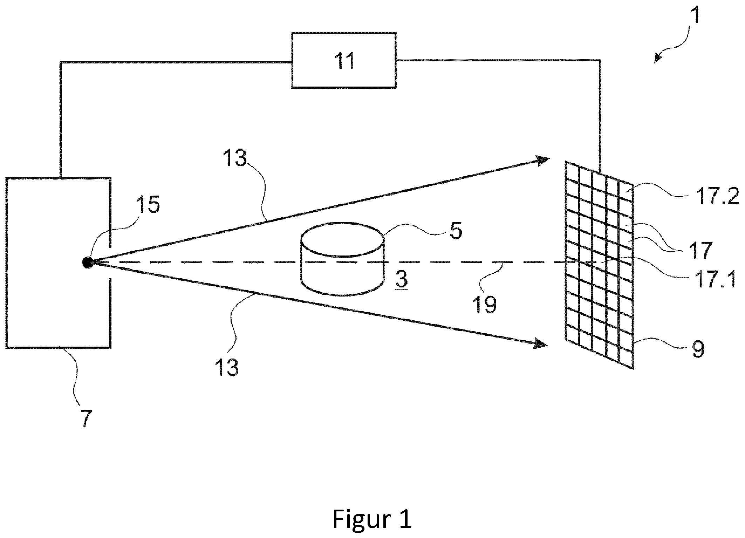

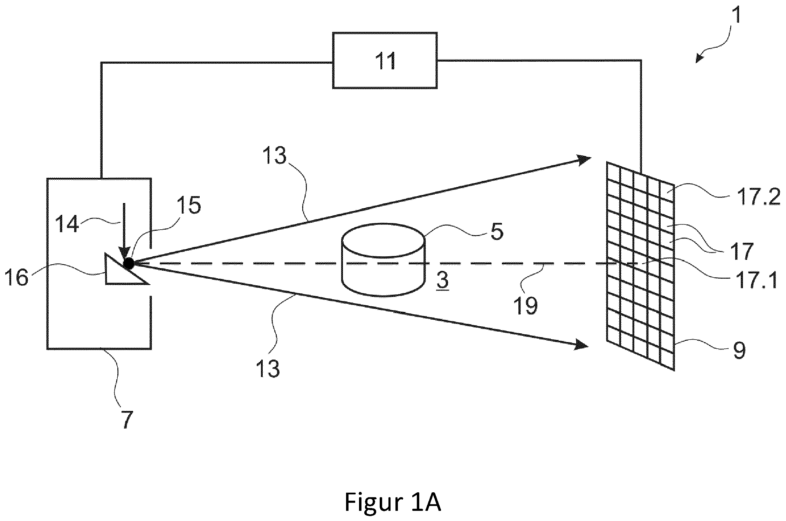

Figur 1 zeigt schematisch eine Abbildungsvorrichtung 1 gemäß einer Ausführungsform im Betrieb beim Durchführen eines Abbildungsverfahrens gemäß einer Ausführungsform. Die Abbildungsvorrichtung 1 weist einen Aufnahmeraum 3 auf, in dem ein abzubildendes Untersuchungsobjekt 5 aufgenommen ist.

Die Abbildungsvorrichtung 1 weist eine Strahlungserzeugungsvorrichtung 7, eine Detektoranordnung 9 und eine Steuervorrichtung 11 auf. Die Steuervorrichtung 11 ist mit der Strahlungserzeugungsvorrichtung 7 und mit der Detektoranordnung 9 verbunden und zum Ansteuern der Strahlungserzeugungsvorrichtung 7 sowie zum Ansteuern und Auslesen der Detektoranordnung 9 ausgebildet.

Die Strahlungserzeugungsvorrichtung 7 ist zum Emittieren von elektromagnetischer Strahlung 13 zum Durchstrahlen des Untersuchungsobjekts 5 ausgehend von einer punktförmigen Strahlungsquellposition 15 ausgebildet. Die elektromagnetische Strahlung 13 wird auch als Untersuchungsstrahlung 13 bezeichnet und liegt als Beispiel in Form von elektromagnetischer Strahlung vor, welche aus Photonen mit Photonenenergien von mindestens 80 keV, bevorzugt mindestens 100 keV, besteht. Die Untersuchungsstrahlung 13 kann z.B. Röntgenstrahlung sein, welche aus Photonen mit Photonenenergien von mindestens 80 keV, bevorzugt mindestens 100 keV, besteht. Die Strahlungserzeugungsvorrichtung 7 ist insbesondere zum Emittieren von Pulsen der Untersuchungsstrahlung 13 ausgehend von der Strahlungsquellposition 15 ausgebildet. Vorliegend ist als Beispiel die Steuervorrichtung 11 zum Ansteuern der Strahlungserzeugungsvorrichtung 7 derart ausgebildet, dass von der Strahlungserzeugungsvorrichtung 7 Pulse der Untersuchungsstrahlung 13 emittiert werden und die Untersuchungsstrahlung 13 somit als gepulste Untersuchungsstrahlung 13 vorliegt, sodass die Strahlungserzeugungsvorrichtung 7 als gepulste Strahlungsquelle fungiert.

Wie in Figur 1A dargestellt, kann die Strahlungserzeugungsvorrichtung 1 zum Beispiel zum Emittieren gepulster Untersuchungsstrahlung 13 ausgebildet sein, indem ein Anregungsstrahl 14 auf ein Target 16 gerichtet wird. Gemäß der Ausführungsform nach Figur 1A kann der Strahlungserzeuger 7 z.B. eine laserbasierte oder elektronenstrahlbasierte Strahlungsquelle sein, bei der mittels Anregung des Materials des Targets 16 mittels eines Anregungsstrahls 14 in Form eines Elektronenstrahls oder Laserstrahls Untersuchungsstrahlung 13 erzeugt wird, wobei die Untersuchungsstrahlung 13 als Beispiel aus Photonen mit Photonenenergien von mindestens 80 keV, bevorzugt mindestens 100 keV, besteht. Bei der Ausführungsform nach Figur 1A ist die Steuervorrichtung 11 zum Ansteuern der Strahlungserzeugungsvorrichtung 7 derart ausgebildet, dass der Anregungsstrahl 14 als gepulster Anregungsstrahl 14 vorliegt und demgemäß auch die Untersuchungsstrahlung 13 als gepulste Untersuchungsstrahlung 13 vorliegt, sodass die Strahlungserzeugungsvorrichtung 7 als gepulste Strahlungsquelle fungiert. Die Strahlungserzeugungsvorrichtung 7 kann jedoch auch auf andere Art und Weise als in Figur 1A veranschaulicht zum Emittieren gepulster Untersuchungsstrahlung 13 ausgebildet sein.

Die Steuervorrichtung 11 ist zum Ansteuern der Strahlungserzeugungsvorrichtung 7 derart ausgebildet, dass von der Strahlungserzeugungsvorrichtung 7 die von der Strahlungsquellposition 15 ausgehende Strahlungsemission alternierend zu vorgegebenen Pulsemissions-Anfangszeitpunkten $t_{PA}^i$ begonnen und zu vorgegebenen Pulsemissions-Endzeitpunkten $t_{PE}^i$ beendet wird, wobei der Index *PA* die Pulsemissions-Anfangszeitpunkte kennzeichnet, der Index PE die Pulsemissions-Endzeitpunkte kennzeichnet, und der Index *i* ein Laufindex ist, welcher die einzelnen Pulse kennzeichnet (so bezeichnet z.B. $t_{PA}^1$ den Pulsemissions-Anfangszeitpunkt und $t_{PE}^1$ den Pulsemissions-Endzeitpunkt des Strahlungspulses mit dem Laufindex *i* = 1, d.h. des ersten Pulses, $t_{PA}^2$ den Pulsemissions-Anfangszeitpunkt und $t_{PE}^2$ den Pulsemissions-Endzeitpunkt des Strahlungspulses mit dem Laufindex *i* = 2, d.h. des zweiten Pulses etc., insbesondere ist *i* eine natürliche Zahl).

Die derart erzeugten Strahlungspulse der Untersuchungsstrahlung 13 verlaufen von der Strahlungsquellposition 15 divergent, hier als Beispiel kegelförmig in Form eines Strahlungskegels, in Richtung zu dem Aufnahmeraum 3 bzw. dem darin angeordneten Untersuchungsobjekt 5, wobei in Figur 1 und Figur 1A die Randstrahlen 13 des erzeugten Strahlungskegels veranschaulicht sind. Das Untersuchungsobjekt 5 wird von der gepulsten Röntgenstrahlung 13 durchstrahlt, wobei die transmittierte Strahlung Rückschlüsse auf die innere Struktur des Untersuchungsobjekts 5 ermöglicht. Die durch das Untersuchungsobjekt 5 hindurch transmittierte Röntgenstrahlung 13 wird mittels der Detektoranordnung 9 erfasst bzw. detektiert.

Die Detektoranordnung 9 weist mehrere separate Detektoren 17 auf, welche auch als Einzeldetektoren 17 bezeichnet werden. Die Einzeldetektoren 17 können vorliegend zum Beispiel als Röntgendetektoren 17 ausgebildet sein. Die Einzeldetektoren 17 sind in Form eines ebenen Detektor-Arrays bzw. einer ebenen Detektor-Matrix mit mehreren Zeilen und mehreren Spalten angeordnet, wobei jede Zeile und jede Spalte mehrere der Einzeldetektoren 17 aufweist. Vorliegend sind die Einzeldetektoren 17 in Form einer ebenen, rechteckförmigen Detektor-Matrix mit elf Zeilen und fünf Spalten angeordnet, sodass innerhalb jeder Zeile fünf Einzeldetektoren 17 und innerhalb jeder Spalte elf Einzeldetektoren 17 vorliegen, wobei die zum Strahlungsempfang vorgesehenen Stirnflächen aller Einzeldetektoren 17 innerhalb einer gemeinsamen ebenen, rechteckigen Fläche liegen. Jeder der Einzeldetektoren 17 ist zum Detektieren der durch das Untersuchungsobjekt 5 hindurchtretenden Untersuchungsstrahlung 13 (z.B. Röntgenstrahlung 13) und zum Erzeugen eines Detektorsignals basierend auf der detektierten Untersuchungsstrahlung 13 ausgebildet. Die Abbildungsvorrichtung 1 ist (z.B. indem die Steuervorrichtung 11 entsprechend ausgebildet ist) basierend auf den Detektorsignalen der Strahlungsdetektoren 17 zum Erzeugen einer Abbildung des Inneren des durchstrahlten Untersuchungsobjekts 5 ausgebildet, z.B. zum Erzeugen einer Röntgenabbildung des Untersuchungsobjekts 5.

Der Strahlungserzeuger 7 und die Detektoranordnung bzw. Detektor-Matrix 9 sind derart angeordnet, dass die Strahlungsquellposition 15 auf der zentralen Normale 19 der Detektor-Matrix 9 angeordnet ist. Die zentrale Normale 19 der Detektor-Matrix ist die Normale bzw. Gerade, welche durch das Zentrum der durch die Detektor-Matrix definierten ebenen, rechteckigen Fläche orthogonal zu derselben verläuft. Die Normale 19 verläuft somit insbesondere durch den zentralen Einzeldetektor 17.1 der Detektor-Anordnung 9. Somit weist die Detektor-Matrix 9 Einzeldetektoren 17 mit unterschiedlichen Abständen zu der Strahlungsquellposition 15 auf. Insbesondere weist von allen Einzeldetektoren 17 der im Zentrum der Detektor-Matrix 9 angeordnete zentrale Einzeldetektor 17.1 den kleinsten Abstand zu der Strahlungsquellposition 15 auf, wohingegen die an den Ecken der Detektor-Matrix 9 angeordneten Einzeldetektoren 17 den größten Abstand zu der Strahlungsquellposition 15 aufweisen (wobei in Figur 1 als Beispiel ein solcher an einer Eckposition bzw. Ecke der Detektor-Matrix 9 angeordneter Einzeldetektor 17 mit dem Bezugszeichen 17.2 gekennzeichnet ist). Der Strahlungserzeuger 7 ist derart ausgebildet, dass der von ihm erzeugte Strahlungskegel 13 die gesamte Detektor-Matrix 9 erfasst, d.h. dass die gesamte Detektor-Matrix 9 mit allen Einzeldetektoren 17 innerhalb des Strahlungskegels angeordnet ist.

Die Steuervorrichtung 11 ist zum Ansteuern der Detektor-Anordnung 9 derart ausgebildet, dass für jeden der Einzeldetektoren 17 die Strahlungsdetektion alternierend zu vorgegebenen Detektions-Anfangszeitpunkten $t_{DA,j}^i$ begonnen und zu vorgegebenen Detektions-Endzeitpunkten $t_{DE,j}^i$ beendet wird, wobei der Zeitraum zwischen einem Detektions-Anfangszeitpunkt und dem darauffolgenden Detektions-Endzeitpunkt eines jeweiligen Einzeldetektors 17 jeweils ein Mess-Zeitfenster des entsprechenden Einzeldetektors 17 bildet. Bei den Bezeichnungen $t_{DA,j}^i$ der Detektions-Anfangszeitpunkte bzw. $t_{DE,j}^i$ der Detektions-Endzeitpunkte kennzeichnet der Index $DA$ die Detektions-Anfangszeitpunkte, der Index $DE$ die Detektions-Endzeitpunkte, der Index $j$ die einzelnen Einzeldetektoren 17 (wobei vorliegend, da die Detektor-Matrix 9 als Beispiel elf Zeilen und fünf Spalten aufweist und somit 55 Einzeldetektoren 17 aufweist, $j$ eine natürliche Zahl ist mit $j$ = 1, 2, 3, ..., 55), und der Laufindex $i$ die einzelnen Mess-Zeitfenster (so bezeichnet z.B. $t_{DA,j}^1$ den Anfangszeitpunkt und $t_{DE,j}^1$ den Endzeitpunkt des Mess-Zeitfensters mit dem Laufindex $i$ = 1, d.h. des ersten Mess-Zeitfensters, für den $j$-ten Einzeldetektor). Nachfolgend wird mit dem Index $j$ = 1 der zentrale Einzeldetektor 17.1 und mit dem Index $j$ = 2 der Einzeldetektor 17.2, der an der in Figur 1 gekennzeichneten Eckposition der Detektor-Matrix 9 angeordnet ist, bezeichnet.

Die Steuervorrichtung 11 kann insbesondere zum Ansteuern jedes der Einzeldetektoren 17 mit vorgegebenen Detektions-Anfangszeitpunkten $t_{DA,j}^i$ und vorgegebenen Detektions-Endzeitpunkten $t_{DE,j}^i$ ausgebildet sein. Die Steuervorrichtung 11 kann insbesondere derart ausgebildet sein, dass von ihr für jeden der Einzeldetektoren 17 entsprechende Detektions-Anfangszeitpunkte $t_{DA,j}^i$ und Detektions-Endzeitpunkte $t_{DE,j}^i$ vorgegeben werden, und dass von ihr die Einzeldetektoren 17 mit den derart vorgegebenen Detektions-Anfangszeitpunkten und Detektions-Endzeitpunkten angesteuert werden. Des Weiteren kann die Steuervorrichtung 11 insbesondere zum Ansteuern jedes der Einzeldetek-

13

toren 17 mit vorgegebenen Detektions-Anfangszeitpunkten $t^i_{DA,j}$ und vorgegebenen Detektions-Endzeitpunkten $t^i_{DE,j}$ derart ausgebildet sein, dass für den jeweiligen Einzeldetektor 17 die Strahlungsdetektion zu den für ihn vorgegebenen Detektions-Anfangszeitpunkten $t^i_{DA,j}$ begonnen und zu den für ihn vorgegebenen Detektions-Endzeitpunkten $t^i_{DE,j}$ beendet wird. Demgemäß kann z.B. vorgesehen sein, dass von der Steuervorrichtung 11 für jeden Einzeldetektor 17 Detektions-Anfangszeitpunkte $t^i_{DA,j}$ und Detektions-Endzeitpunkte $t^i_{DE,j}$ vorgegeben werden und die Steuervorrichtung 11 zum Ansteuern der Detektoranordnung 9 derart ausgebildet ist, dass jeder der Einzeldetektoren 17 die für ihn vorgegebenen Detektions-Anfangszeitpunkte und Detektions-Endzeitpunkte aufweist (d.h. zu den für den jeweiligen Einzeldetektor vorgegebenen Detektions-Anfangszeitpunkten in den aktiven Zustand übergeht und zu den für den jeweiligen Einzeldetektor vorgegebenen Detektions-Endzeitpunkten in den passiven Zustand übergeht).

Die Steuervorrichtung 11 ist zum Ansteuern der Detektor-Anordnung 9 derart ausgebildet, dass nicht alle Einzeldetektoren 17 der Detektor-Matrix 9 die gleichen Detektions-Endzeitpunkte $t^i_{DE,j}$ aufweisen. Die Steuervorrichtung 11 kann insbesondere zum Ansteuern der Detektor-Anordnung 9 derart ausgebildet sein, dass nicht alle Einzeldetektoren 17 der Detektor-Matrix 9 mit den gleichen Detektions-Endzeitpunkten $t^i_{DE,j}$ angesteuert werden, sondern zumindest einige der Einzeldetektoren 17 mit unterschiedlichen Detektions-Endzeitpunkten $t^i_{DE,j}$ angesteuert werden. Es kann somit insbesondere vorgesehen sein, dass (z.B. von der Steuervorrichtung 11) zumindest für einige der Einzeldetektoren 17 unterschiedliche Detektions-Endzeitpunkte $t^i_{DE,j}$ vorgegeben sind bzw. werden, und dass diese Einzeldetektoren 17 mit den vorgegebenen unterschiedlichen Detektions-Endzeitpunkten $t^i_{DE,j}$ derart angesteuert werden, dass sie diese vorgegebenen unterschiedlichen Detektions-Endzeitpunkte $t^i_{DE,j}$ aufweisen (d.h. zu diesen vorgegebenen unterschiedlichen Detektions-Endzeitpunkten $t^i_{DE,j}$ vom aktiven in den passiven Zustand wechseln).

Die Steuervorrichtung 11 ist insbesondere zum Ansteuern der Detektor-Anordnung 9 derart ausgebildet, dass Einzeldetektoren 17 mit unterschiedlichen Abständen zu der Strahlungsquellposition 15 mit unterschiedlichen Detektions-Endzeitpunkten $t^i_{DE,j}$ angesteuert werden, wobei die Detektions-Endzeitpunkte für Einzeldetektoren 17 mit einem größeren Abstand zu der Strahlungsquellposition 15 zeitlich hinter den Detektions-Endzeitpunkten für Einzeldetektoren 17 mit einem demgegenüber kleineren Abstand zu der Strahlungsquellposition 15 liegen. Somit wird ein jeweiliger Einzeldetektor 17 umso später vom aktiven in den passiven Zustand geschaltet, je größer sein Abstand von der Strahlungsquellposition 15 ist.

Alle Einzeldetektoren 17, welche den gleichen Abstand zu der Strahlungsquellposition 15 aufweisen, werden von der Steuervorrichtung 11 mit denselben Detektions-Endzeitpunkten $t^i_{DE,j}$ angesteuert, vorliegend z.B. die vier an den Eckpositionen der Detektor-Matrix 9 angeordneten Einzeldetektoren.

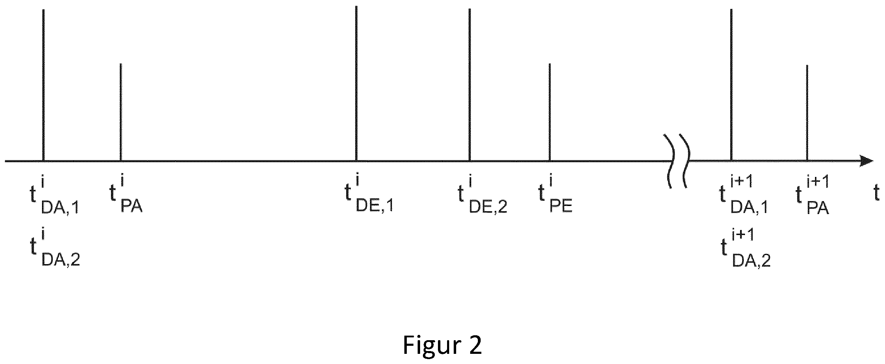

Bezüglich der Detektions-Anfangszeitpunkte $t^i_{DA,j}$ werden vorliegend als Beispiel alle Einzeldetektoren 17 mit denselben Detektions-Anfangszeitpunkten $t^i_{DA,j}$ angesteuert, d.h. jeweils zu denselben Zeitpunkten vom passiven in den aktiven Zustand geschaltet, sodass gilt $t^i_{DA,j} = t^i_{DA,k}$ für alle $i$ und alle $j$, $k$ = 1, 2, 3, ..., 55 (wie in Figur 2 exemplarisch für die Einzeldetektoren 17.1 und 17.2 mit den Indizes $j$ = 1 bzw. $j$ = 2 veranschaulicht). Alternativ dazu kann bezüglich der Detektions-Anfangszeitpunkte $t^i_{DA,j}$ vorgesehen sein, dass zumindest die Einzeldetektoren 17 mit unterschiedlichen Detektions-Endzeitpunkten (d.h. die Einzeldetektoren 17 mit unterschiedlichen Abständen zu der Strahlungsquellposition 15) von der Steuervorrichtung 11 auch mit unterschiedlichen Detektions-Anfangszeitpunkten $t^i_{DA,j}$ angesteuert werden, d.h. zu unterschiedlichen Zeitpunkten vom passiven in den aktiven Zustand geschaltet werden (nicht dargestellt).

In Figur 2 ist anhand eines Zeitstrahls (wobei $t$ die Zeit bezeichnet) exemplarisch die zeitliche Abfolge der

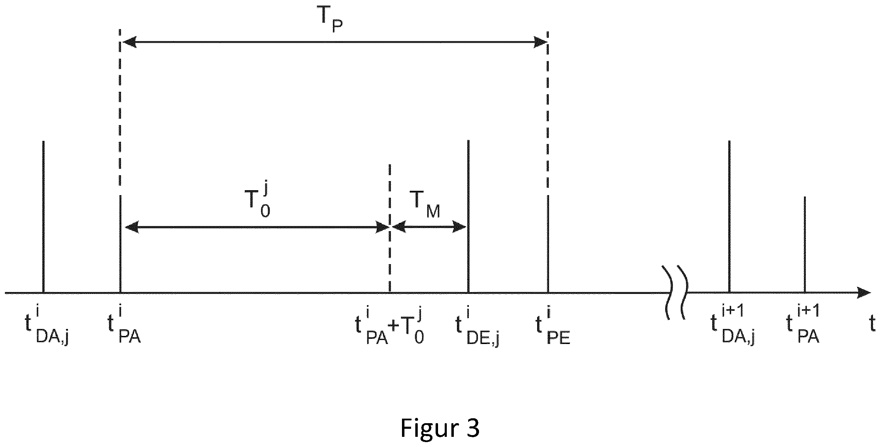

Detektions-Anfangszeitpunkte $t^i_{DA,1}$ und der Detektions-Endzeitpunkte $t^i_{DE,1}$ des dem Index $j = 1$ zugeordneten zentralen Einzeldetektors 17.1 und der Detektions-Anfangszeitpunkte $t^i_{DA,2}$ und der Detektions-Endzeitpunkte $t^i_{DE,2}$ des dem Index $j = 2$ zugeordneten Einzeldetektors 17.2, der an der Eckposition der Detektor-Matrix 9 angeordnet ist, bezüglich des Pulsemissions-Anfangszeitpunktes $t^i_{PA}$ und des Pulsemissions-Endzeitpunktes $t^i_{PE}$ eines ersten Strahlungspulses mit dem Laufindex $i$ und bezüglich des Pulsemissions-Anfangszeitpunktes $t^{i+1}_{PA}$ eines zweiten Strahlungspulses mit dem Laufindex $i + 1$ dargestellt (wobei z.B. $i = 1$ sein kann). Zur besseren Unterscheidbarkeit sind in Figur 2 und Figur 3 die Detektions-Anfangszeitpunkte und Detektions-Endzeitpunkte mit längeren Strichen und die Pulsemissions-Anfangszeitpunkte und Pulsemissions-Endzeitpunkte mit (demgegenüber) kürzeren Strichen gekennzeichnet. Wie aus Figur 2 ersichtlich, werden die Einzeldetektoren 17.1 ($j = 1$) und 17.2 ($j = 2$) mit denselben Detektions-Anfangszeitpunkten angesteuert, d.h. $t^i_{DA,1} = t^i_{DA,2}$ für alle $i$. Wie ebenfalls aus Figur 2 ersichtlich, liegen zudem die Detektions-Endzeitpunkte $t^i_{DE,2}$ des weiter von der Strahlungsquellposition 15 entfernten Einzeldetektors 17.2 ($j = 2$) zeitlich hinter den Detektions-Endzeitpunkte $t^i_{DE,1}$ des (demgegenüber) näher an der Strahlungsquellposition 15 angeordneten zentralen Einzeldetektors 17.1 ($j = 1$), d.h. $t^i_{DE,1} < t^i_{DE,2}$ für alle $i$. Demgemäß erfolgt die Ansteuerung der Einzeldetektoren 17 vorliegend insbesondere derart, dass die Detektions-Endzeitpunkte $t^i_{DE,1}$ für den zentralen Einzeldetektor 17.1 (der von allen Einzeldetektoren den kleinsten Abstand zu der Strahlungsquellposition 15 aufweist) zeitlich vor den Detektions-Endzeitpunkten $t^i_{DE,2}$ für die an den Eckpositionen der Detektor-Matrix 9 angeordneten Einzeldetektoren 17.2 (die von allen Einzeldetektoren den größten Abstand zu der Strahlungsquellposition 15 aufweisen) liegen.

In Figur 3 ist anhand eines Zeitstrahls (wobei $t$ die Zeit bezeichnet) die zeitliche Abfolge der Detektions-Anfangszeitpunkte $t^i_{DA,j}$ und Detektions-Endzeitpunkte $t^i_{DE,j}$ eines jeweiligen, dem Index j zugeordneten Einzeldetektors 17 bezüglich der Pulsemissions-Anfangszeitpunkte $t^i_{PA}$ und Pulsemissions-Endzeitpunkte $t^i_{PE}$ veranschaulicht.

Mittels der Steuervorrichtung 11 werden die Einzeldetektoren 17 der Detektoranordnung 9 in zeitlicher Abstimmung auf die Taktung der gepulsten Strahlungsquelle 7 alternierend aktiv und passiv geschaltet, wobei jedem Strahlungspuls genau ein Mess-Zeitfenster jedes Einzeldetektors 17 zugeordnet wird. Gemäß der vorstehend erläuterten Notation wird dem Strahlungspuls mit dem Pulsemissions-Anfangszeitpunkt $t^i_{PA}$ und dem Pulsemissions-Endzeitpunkt $t^i_{PE}$ für den mittels des Laufindex $j$ gekennzeichneten Einzeldetektor 17 das Mess-Zeitfenster mit dem Detektions-Anfangszeitpunkt $t^i_{DA,j}$ und dem Detektions-Endzeitpunkt $t^i_{DE,j}$ zugeordnet, d.h. ein Strahlungspuls und das diesem Strahlungspuls zugeordnete Mess-Zeitfenster jedes Einzeldetektors 17 weisen denselben Laufindex $i$ auf. Zwischen zwei aufeinanderfolgenden Pulsemissions-Anfangszeitpunkten $t^i_{PA}$ und $t^{i+1}_{PA}$ liegt für jeden Einzeldetektor 17 jeweils ein einziger Detektions-Endzeitpunkt. Zwischen zwei aufeinanderfolgenden Pulsemissions-Anfangszeitpunkten $t^i_{PA}$ und $t^{i+1}_{PA}$ liegt zudem für jeden Einzeldetektor 17 jeweils ein einziger Detektions-Anfangszeitpunkt.

Dabei liegt für jeden, einem jeweiligen Index $j$ zugeordneten Einzeldetektor 17 der Detektions-Endzeitpunkt $t^i_{DE,j}$ des einem jeweiligen Strahlungspuls mit dem Index $i$ zugeordneten Mess-Zeitfensters zeitlich hinter dem Pulsemissions-Anfangszeitpunkt $t^i_{PA}$ des betreffenden Strahlungspulses, d.h. es gilt $t^i_{DE,j} > t^i_{PA}$ (für alle $i$ und alle $j$). Zudem liegt für jeden Einzeldetektor 17 mit Index $j$ der Detektions-Endzeitpunkt $t^i_{DE,j}$ des einem jeweiligen Strahlungspuls mit dem Index $i$ zugeordneten Mess-Zeitfensters zeitlich vor dem Pulsemissions-Anfangszeitpunkt $t^{i+1}_{PA}$ des nach-

folgenden Strahlungspulses, d.h. es gilt $t_{DE,j}^i < t_{PA}^{i+1}$ . Darüber hinaus ist die Steuervorrichtung 11 zum Ansteuern der Detektoranordnung 9 derart ausgebildet, dass für jeden, einem jeweiligen Index $j$ zugeordneten Einzeldetektor 17 der Detektions-Endzeitpunkt $t_{DE,j}^i$ des einem jeweiligen Strahlungspuls mit dem Index $i$ zugeordneten Mess-Zeitfensters zeitlich vor dem Pulsemissions-Endzeitpunkt des betreffenden Strahlungspulses liegt, d.h. $t_{DE,j}^i < t_{PE}^i$ .

Die Steuervorrichtung 11 ist zudem zum Ansteuern der Detektoranordnung 9 derart ausgebildet, dass für jeden Einzeldetektor 17 das einem jeweiligen Strahlungspuls mit dem Index $i$ zugeordnete Mess-Zeitfenster zeitlich vor dem Pulsemissions-Anfangszeitpunkt $t_{PA}^i$ des betreffenden Pulses beginnt, d.h. es gilt $t_{DA,j}^i < t_{PA}^i$ (für alle $i$ und alle $j$). Zudem beginnt für jeden Einzeldetektor 17 das einem jeweiligen Strahlungspuls mit dem Index $i$ zugeordnete Mess-Zeitfenster zeitlich nach dem Pulsemissions-Endzeitpunkt $t_{PE}^{i-1}$ des vorhergehenden Strahlungspulses, d.h. es gilt $t_{DA,j}^i > t_{PE}^{i-1}$ bzw. $t_{DA,j}^{i+1} > t_{PE}^i$ .

Somit gilt vorliegend $t_{DA,j}^i < t_{PA}^i < t_{DE,j}^i < t_{PE}^i < t_{DA,j}^{i+1} < t_{PA}^{i+1}$ für alle Indizes $i$ sowie für alle Indizes $j$ bzw. für jeden Einzeldetektor 17. Demgemäß liegt insbesondere jeder Pulsemissions-Anfangszeitpunkt $t_{PA}^i$ innerhalb des dem entsprechenden Puls zugeordneten Mess-Zeitfensters $[t_{DA,j}^i; t_{DE,j}^i]$ . Zudem ist für jeden Einzeldetektor 17 zwischen zwei aufeinanderfolgenden Pulsemissions-Anfangszeitpunkten $t_{PA}^i$ und $t_{PA}^{i+1}$ jeweils ein (einziger) Detektions-Endzeitpunkt $t_{DE,j}^i$ und ein (einziger) Detektions-Anfangszeitpunkt $t_{DA,j}^{i+1}$ derart angeordnet, dass der Detektions-Anfangszeitpunkt $t_{DA,j}^{i+1}$ zeitlich hinter dem Detektions-Endzeitpunkt $t_{DE,j}^i$ liegt (wobei der Detektions-Endzeitpunkt $t_{DE,j}^i$ und der Detektions-Anfangszeitpunkt $t_{DA,j}^{i+1}$ zu unterschiedlichen Mess-Zeitfenstern gehören, nämlich zu dem Mess-Zeitfenster zu dem Index $i$ einerseits und dem Mess-Zeitfenster zu dem Index $i + 1$ andererseits).

Die Zeit, welche Untersuchungsstrahlung von der Strahlungsquellposition 15 aus bis zum Erreichen eines jeweiligen, dem Index $j$ zugeordneten Einzeldetektors 17 benötigt, wird auch als Laufzeit oder Strahlungs-Laufzeit $T_j$ bezeichnet. Die Zeit, welche ungestreute Untersuchungsstrahlung von der Strahlungsquellposition 15 aus bis zum Erreichen eines jeweiligen, dem Index $j$ zugeordneten Einzeldetektors 17 benötigt, wird auch als Direkt-Laufzeit $T_j^0$ bezeichnet. Die Direkt-Laufzeit $T_j^0$ , welche ungestreute Untersuchungsstrahlung von der Strahlungsquellposition 15 aus bis zum Erreichen des dem Index $j$ zugeordneten Einzeldetektors 17 benötigt, entspricht der Zeit, welche zum Zurücklegen des Abstandes $a_j$ zwischen der Strahlungsquellposition 15 und dem jeweiligen, dem Index $j$ zugeordneten Einzeldetektor 17 mit Lichtgeschwindigkeit $c$ benötigt wird (wobei die im Vakuum vorliegende Lichtgeschwindigkeit verwendet werden kann). Die Direkt-Laufzeit $T_j^0$ entspricht somit dem Quotienten aus dem Abstand $a_j$ des jeweiligen Einzeldetektors 17 mit dem Index $j$ von der Strahlungsquellposition 15 und der Lichtgeschwindigkeit $c$, d.h. $T_j^0 = a_j/c$ .

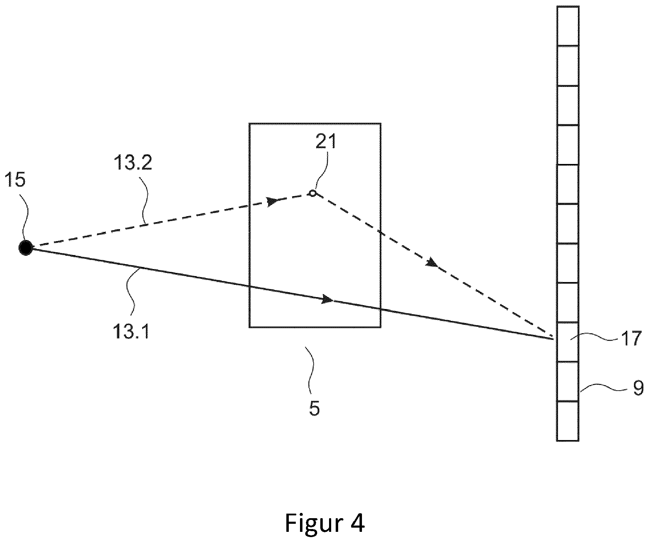

Figur 4 zeigt exemplarisch den Verlauf ungestreuter Untersuchungsstrahlung 13.1 und gestreuter Untersuchungsstrahlung 13.2 zwischen der Strahlungsquellposition 15 und einem jeweiligen Einzeldetektor 17. Ungestreute Untersuchungsstrahlung 13.1 gelangt geradlinig und somit auf kürzestem Weg direkt von der Strahlungsquellposition 15 zu dem jeweiligen Einzeldetektor 17. Gestreute Untersuchungsstrahlung 13.2, welche aufgrund eines als Beispiel an einer Streuposition 21 stattfindenden Streuprozesses (z.B. aufgrund von Compton-Streuung) eine Richtungsänderung erfährt und so zu demselben Einzeldetektor 17 umgelenkt wird, durchläuft einen längeren Weg bis zum Erreichen dieses Einzeldetektors 17, und benötigt hierfür somit eine längere Laufzeit als ungestreute Untersuchungsstrahlung 13.1.

Die Einzeldetektoren 17 werden derart angesteuert, dass die Zeitspanne zwischen dem Eintreffen der ersten (und somit ungestreuten) Strahlungsanteile eines jeweiligen Pulses und dem Detektions-Endzeitpunkt des dem Puls zugeordneten Mess-Zeitfensters für alle Einzeldetektoren 17 (d.h. alle Indizes $j$) gleich groß ist und einem vorgegebenen Wert $T_M$ entspricht. Die ersten Strahlungsanteile eines Strahlungspulses mit dem Pulsemissions-Anfangszeitpunkt $t_{PA}^i$ treffen an einem jeweiligen, dem Index $j$ zugeordneten Einzeldetektor 17 zu der Zeit $t_{PA}^i + T_j^0$ ein, d.h. eine Direkt-Laufzeit $T_j^0$ nach dem Pulsemissions-Anfangszeitpunkt $t_{PA}^i$. Die Zeitspanne $T_M^j$ zwischen dem Eintreffen der ersten Strahlungsanteile eines Strahlungspulses mit dem Pulsemissions-Anfangszeitpunkt $t_{PA}^i$ an einem jeweiligen, dem Index $j$ zugeordneten Einzeldetektor und dem zugeordneten Detektions-Endzeitpunkt $t_{DE,j}^i$ ergibt sich somit zu $T_M^j = t_{DE,j}^i - \left(t_{PA}^i + T_j^0\right) = \left(t_{DE,j}^i - t_{PA}^i\right) - T_j^0$ und entspricht somit dem zeitlichen Abstand zwischen dem Pulsemissions-Anfangszeitpunkt $t_{PA}^i$ des Pulses und dem (darauffolgenden) Detektions-Endzeitpunkt $t_{DE,j}^i$, verringert um die Direkt-Laufzeit $T_j^0$, welche ungestreute Strahlungsanteile ausgehend von der Strahlungsquellposition 15 bis zum Erreichen des dem Index $j$ zugeordneten Einzeldetektors 17 benötigen. Die Einzeldetektoren 17 werden von der Steuervorrichtung 11 derart angesteuert, dass die Zeitspanne $T_M^j$ zwischen dem Eintreffen der ersten Strahlungsanteile eines jeweiligen Pulses und dem Detektions-Endzeitpunkt des dem Puls zugeordneten Mess-Zeitfensters für alle Einzeldetektoren gleich groß ist und dem vorgegebenen Wert $T_M$ entspricht, d.h. $T_M^j = T_M$ für alle $j$. In Figur 3 sind zur Veranschaulichung die Direkt-Laufzeit $T_j^0$ und die Zeitspanne $T_M$ dargestellt.

Die Steuervorrichtung 11 ist zum Ansteuern des Strahlungserzeugers 7 derart ausgebildet, dass von dem Strahlungserzeuger 7 Strahlungspulse mit einer Pulsdauer $T_P$ erzeugt werden, d.h. $t_{PE}^i - t_{PA}^i = T_P$ für alle $i$. Die Steuervorrichtung 11 ist zudem zum Ansteuern der Detektoranordnung 9 derart ausgebildet, dass für jeden Einzeldetektor 17 (bzw. jeden Index $j$) der zeitliche Abstand zwischen einem Pulsemissions-Anfangszeitpunkt $t_{PA}^i$ und dem darauf folgenden Detektions-Endzeitpunkt $t_{DE,j}^i$ kleiner ist als die Summe aus der Pulsdauer $T_P$ und der Direkt-Laufzeit $T_j^0$, welche ungestreute Strahlungsanteile ausgehend von der Strahlungsquellposition 15 bis zum Erreichen des dem Index $j$ zugeordneten Einzeldetektors 17 benötigen, d.h. $t_{DE,j}^i - t_{PA}^i < T_P + T_j^0$ für alle $i$ und alle $j$. Dadurch kann der Streustrahlungsanteil der von den Einzeldetektoren 17 generierten Detektorsignale reduziert werden.

Die Ansteuerung der Detektoranordnung 9 erfolgt zudem derart, dass für jeden Einzeldetektor 17 (bzw. jeden Index $j$) der zeitliche Abstand zwischen einem Pulsemissions-Anfangszeitpunkt $t_{PA}^i$ und dem darauf folgenden Detektions-Endzeitpunkt $t_{DE,j}^i$ kleiner ist als das Doppelte der Direkt-Laufzeit $T_j^0$, welche ungestreute Strahlungsanteile ausgehend von der Strahlungsquellposition 15 bis zum Erreichen des dem Index $j$ zugeordneten Einzeldetektors 17 benötigen, d.h. $t_{DE,j}^i - t_{PA}^i < 2 \cdot T_j^0$ für alle $i$ und alle $j$. Als Beispiel kann vorgesehen sein, dass für jeden Einzeldetektor 17 (bzw. jeden Index $j$) der zeitliche Abstand zwischen einem Pulsemissions-Anfangszeitpunkt $t_{PA}^i$ und dem darauf folgenden Detektions-Endzeitpunkt $t_{DE,j}^i$ kleiner ist als das 1,5-fache der Direkt-Laufzeit $T_j^0$, d.h. $t_{DE,j}^i - t_{PA}^i < 1{,}5 \cdot T_j^0$. Es kann z.B. vorgesehen sein, dass der zeitliche Abstand zwischen einem Pulsemissions-Anfangszeitpunkt $t_{PA}^i$ und dem darauf folgenden Detektions-Endzeitpunkt $t_{DE,j}^i$ kleiner ist als das 1,2-fache der Direkt-Laufzeit $T_j^0$, d.h. $t_{DE,j}^i - t_{PA}^i < 1{,}2 \cdot T_j^0$. Dadurch kann z.B. die Detektion gestreuter Photonen effektiv unterdrückt und auf entsprechend schwach gestreute Photonen beschränkt werden.

Die Steuervorrichtung 11 ist zudem zum Ansteuern der Detektoranordnung 9 derart ausgebildet, dass für jeden Einzeldetektor 17 (bzw. jeden Index $j$) der zeitliche Abstand zwischen einem Pulsemissions-Endzeitpunkt $t_{PE}^i$ und dem darauffolgenden Detektions-Anfangszeitpunkt $t_{DA,j}^{i+1}$ größer ist als das Doppelte der Direkt-Laufzeit $T_j^0$ , welche ungestreute Strahlungsanteile ausgehend von der Strahlungsquellposition 15 bis zum Erreichen des dem Index $j$ zugeordneten Einzeldetektors 17 benötigen, d.h. $t_{DA,j}^{i+1} - t_{PE}^i > 2 \cdot T_j^0$ für alle $i$ und alle $j$. Als Beispiel kann vorgesehen sein, dass für jeden Einzeldetektor 17 (bzw. jeden Index $j$) der zeitliche Abstand zwischen einem Pulsemissions-Endzeitpunkt $t_{PE}^i$ und dem darauffolgenden Detektions-Anfangszeitpunkt $t_{DA,j}^{i+1}$ größer ist als das 5-fache der Direkt-Laufzeit $T_j^0$ , d.h. $t_{DA,j}^{i+1} - t_{PE}^i > 5 \cdot T_j^0$ . Dadurch kann z.B. eine Verfälschung der einem Strahlungspuls zugeordneten, in einem Mess-Zeitfenster generierten Detektorsignale durch Streustrahlung des vorhergehenden Strahlungspulses vermindert oder verhindert werden.

Darüber hinaus kann die Steuervorrichtung 11 zudem zum Ansteuern der Detektoranordnung 9 derart ausgebildet sein, dass für jeden Einzeldetektor 17 (bzw. jeden Index $j$) der zeitliche Abstand zwischen einem Pulsemissions-Endzeitpunkt $t_{PE}^i$ und dem darauffolgenden Detektions-Anfangszeitpunkt $t_{DA,j}^{i+1}$ größer ist als die Pulsdauer $T_P$ der Strahlungspulse, d.h. $t_{DA,j}^{i+1} - t_{PE}^i > T_P$ . Als Beispiel kann vorgesehen sein, dass für jeden Einzeldetektor 17 (bzw. jeden Index $j$) der zeitliche Abstand zwischen einem Pulsemissions-Endzeitpunkt $t_{PE}^i$ und dem darauffolgenden Detektions-Anfangszeitpunkt $t_{DA,j}^{i+1}$ größer ist als das Doppelte der Pulsdauer $T_P$, d.h. $t_{DA,j}^{i+1} - t_{PE}^i > 2 \cdot T_P$ . Es kann z.B. vorgesehen sein, dass der zeitliche Abstand zwischen einem Pulsemissions-Endzeitpunkt $t_{PE}^i$ und dem darauffolgenden Detektions-Anfangszeitpunkt $t_{DA,j}^{i+1}$ größer ist als das 5-fache der Pulsdauer $T_P$, d.h. $t_{DA,j}^{i+1} - t_{PE}^i > 5 \cdot T_P$ .

Die Abbildungsvorrichtung 1 kann z.B. derart ausgebildet sein, dass der maximale Abstand zwischen der Strahlungsquellposition 15 und einem der Einzeldetektoren 17, d.h. der Abstand zwischen der Strahlungsquellposition und dem an der Eckposition angeordneten Einzeldetektor 17.2, als Beispiel 1 m beträgt. In diesem Fall würde die maximale Direkt-Laufzeit ca. $T_2^0 \approx 1m/(3 \cdot 10^8 m/s) \approx 3{,}3\,ns$ betragen (wobei ns für Nano-Sekunde steht). Zudem kann die Abbildungsvorrichtung 1 als Beispiel zum Verwenden von Pulsen der Pulsdauer $T_P$ = 10 $ns$ ausgebildet sein, sodass die Pulsdauer $T_P$ größer ist als die maximale Direkt-Laufzeit, insbesondere größer als das Doppelte der maximalen Direkt-Laufzeit.

**Liste der verwendeten Bezugszeichen**

1      Abbildungsvorrichtung
3      Aufnahmeraum zum Aufnehmen eines Untersuchungsobjekts
5      Untersuchungsobjekt
7      Strahlungserzeugungsvorrichtung
9      Detektoranordnung / Detektor-Matrix
11     Steuervorrichtung
13     elektromagnetische Strahlung / Untersuchungsstrahlung
13.1     ungestreute Untersuchungsstrahlung
13.2     gestreute Untersuchungsstrahlung
14     Anregungsstrahl
15     Strahlungsquellposition
16     Target / Targetmaterial
17     Detektor / Einzeldetektor / Röntgendetektor
17.1     zentraler Einzeldetektor
17.2     an Eckposition der Detektor-Matrix angeordneter Einzeldetektor

19     Zentral-Normale der Detektor-Matrix

21     Streuposition

$t_{PA}^{i}$     Pulsemissions-Anfangszeitpunkt eines dem Laufindex *i* zugeordneten Pulses

$t_{PE}^{i}$     Pulsemissions-Endzeitpunkt eines dem Laufindex *i* zugeordneten Pulses

$t_{DA,j}^{i}$     Detektions-Anfangszeitpunkt eines dem Index *j* zugeordneten Einzeldetektors

$t_{DE,j}^{i}$     Detektions-Endzeitpunkt eines dem Index *j* zugeordneten Einzeldetektors

$T_{j}^{0}$     Laufzeit zum Zurücklegen des Abstandes zwischen der Strahlungsquellposition und dem Einzeldetektor mit dem Index *j* mit Lichtgeschwindigkeit

$T_{M}$     Zeitspanne zwischen dem Eintreffen der ersten Strahlungsanteile eines Pulses an einem Einzeldetektor und dem zugeordneten Detektions-Endzeitpunkt

$T_{P}$     Pulsdauer der Strahlungspulse der Untersuchungsstrahlung

**Patentansprüche**

1. Vorrichtung (1) zur Transmissionsbildgebung zum Abbilden des Inneren eines Untersuchungsobjekts (5), aufweisend:

   - eine Strahlungserzeugungsvorrichtung (7) zum Emittieren von Pulsen elektromagnetischer Strahlung (13) zum Durchstrahlen des Untersuchungsobjekts (5) ausgehend von einer Strahlungsquellposition (15),
   - eine Detektoranordnung (9) mit mehreren Detektoren (17) zum Detektieren der Strahlung, und
   - eine Steuervorrichtung (11) die zum Ansteuern der Detektoranordnung (9) derart ausgebildet ist, dass für jeden der Detektoren (17) die Strahlungsdetektion alternierend zu vorgegebenen Detektions-Anfangszeitpunkten ( $t_{DA,j}^{i}$ ) begonnen und zu vorgegebenen Detektions-Endzeitpunkten ( $t_{DE,j}^{i}$ ) beendet wird, wobei die Steuervorrichtung (11) zum Ansteuern der Detektoranordnung (9) derart ausgebildet ist, dass zumindest einige der Detektoren (17.1, 17.2) mit unterschiedlichen Detektions-Endzeitpunkten ( $t_{DE,1}^{i}, t_{DE,2}^{i}$ ) angesteuert werden und unterschiedliche Detektions-Endzeitpunkte ( $t_{DE,1}^{i}, t_{DE,2}^{i}$ ) aufweisen, **dadurch gekennzeichnet, dass**

   - die Detektoren (17.1, 17.2), welche unterschiedliche Detektions-Endzeitpunkte ( $t_{DE,1}^{i}, t_{DE,2}^{i}$ ) aufweisen, unterschiedliche Abstände zu der Strahlungsquellposition (15) aufweisen.

2. Vorrichtung nach Anspruch 1, wobei die Steuervorrichtung (11) zum Ansteuern der Detektoranordnung (9) derart ausgebildet ist, dass die Detektions-Endzeitpunkte ( $t_{DE,2}^{i}$ ) für Detektoren (17.2) mit einem größeren Abstand zu der Strahlungsquellposition (15) zeitlich hinter den Detektions-Endzeitpunkten ( $t_{DE,1}^{i}$ ) für Detektoren (17.1) mit einem demgegenüber kleineren Abstand zur Strahlungsquellposition (15) liegen.

3. Vorrichtung nach einem der Ansprüche 1 und 2, wobei

   - die Strahlungserzeugungsvorrichtung (7) zum Emittieren von Pulsen elektromagnetischer Strahlung (13) derart ausgebildet ist, dass die von der Strahlungsquellposition (15) ausgehende Strahlungsemission alternierend zu vorgegebenen Pulsemissions-Anfangszeitpunkten ( $t_{PA}^{i}$ ) begonnen und zu vorgegebenen Pulsemissions-Endzeitpunkten ( $t_{PE}^{i}$ ) beendet wird, und

   - die Steuervorrichtung (11) zum Ansteuern der Detektoranordnung (9) derart ausgebildet ist, dass für jeden Detektor (17) zwischen zwei aufeinanderfolgenden Pulsemissions-Anfangszeitpunkten ( $t_{PA}^{i}$, $t_{PA}^{i+1}$ ) jeweils ein Detektions-Anfangszeitpunkt ( $t_{DA,j}^{i+1}$ ) und ein Detektions-Endzeitpunkt ( $t_{DE,j}^{i}$ ) derart vorliegen, dass der Detektions-Anfangszeitpunkt ( $t_{DA,j}^{i+1}$ ) zeitlich hinter dem Detektions-Endzeitpunkt ( $t_{DE,j}^{i}$ ) liegt.

4.  Vorrichtung nach einem der Ansprüche 1 bis 3, wobei die Steuervorrichtung (11) zum Ansteuern der Detektoranordnung (9) derart ausgebildet ist, dass alle Detektoren (17) dieselben Detektions-Anfangszeitpunkte ( $t_{DA,j}^{i}$ ) aufweisen.

5.  Vorrichtung nach einem der Ansprüche 1 bis 4, wobei die Steuervorrichtung (11) zum Ansteuern der Detektoranordnung (9) derart ausgebildet ist, dass die Differenz zwischen dem zeitlichen Abstand, der zwischen einem Pulsemissions-Anfangszeitpunkt ( $t_{PA}^{i}$ ) und dem darauf folgenden Detektions-Endzeitpunkt ( $t_{DE,j}^{i}$ ) eines jeweiligen Detektors vorliegt, und der Zeit ( $T_{j}^{0}$ ), welche zum Zurücklegen des Abstandes zwischen der Strahlungsquellposition (15) und dem jeweiligen Detektor mit Lichtgeschwindigkeit erforderlich ist, für alle Detektoren gleich groß ist.

6.  Vorrichtung nach einem der Ansprüche 1 bis 5, wobei die Steuervorrichtung (11) zum Ansteuern der Detektoranordnung (9) derart ausgebildet ist, dass für jeden Detektor (17) der zeitliche Abstand zwischen einem Pulsemissions-Anfangszeitpunkt ( $t_{PA}^{i}$ ) und dem darauf folgenden Detektions-Endzeitpunkt ( $t_{DE,j}^{i}$ ) jeweils kleiner ist als die Summe aus der Pulsdauer ($T_P$) und der Zeit ( $T_{j}^{0}$ ), welche zum Zurücklegen des Abstandes zwischen der Strahlungsquellposition (15) und dem jeweiligen Detektor (17) mit Lichtgeschwindigkeit erforderlich ist.

7.  Vorrichtung nach einem der Ansprüche 1 bis 6, wobei die Steuervorrichtung (11) zum Ansteuern der Detektoranordnung (9) derart ausgebildet ist, dass für jeden Detektor der zeitliche Abstand zwischen einem Pulsemissions-Anfangszeitpunkt ( $t_{PA}^{i}$ ) und dem darauf folgenden Detektions-Endzeitpunkt ( $t_{DE,j}^{i}$ ) kleiner ist als das Doppelte der Zeit ( $T_{j}^{0}$ ), welche zum Zurücklegen des Abstandes zwischen der Strahlungsquellposition (15) und dem jeweiligen Detektor (17) mit Lichtgeschwindigkeit erforderlich ist.

8.  Vorrichtung nach einem der Ansprüche 1 bis 7, wobei die Steuervorrichtung (11) zum Ansteuern der Detektoranordnung (9) derart ausgebildet ist, dass für jeden Detektor (17) der zeitliche Abstand zwischen einem Pulsemissions-Endzeitpunkt ( $t_{PE}^{i}$ ) und dem darauf folgenden Detektions-Anfangszeitpunkt ( $t_{DA,j}^{i+1}$ ) größer ist als das Doppelte der Zeit ( $T_{j}^{0}$ ), welche zum Zurücklegen des Abstandes zwischen der Strahlungsquellposition (15) und dem jeweiligen Detektor (17) mit Lichtgeschwindigkeit erforderlich ist.

9.  Vorrichtung nach einem der Ansprüche 1 bis 8, wobei die Strahlungserzeugungsvorrichtung (7) zum Erzeugen von Pulsen elektromagnetischer Strahlung (13) ausgebildet ist, die Photonen mit Photonenenergien von mindestens 80 keV aufweisen oder daraus bestehen.

10. Verfahren zur Transmissionsbildgebung zum Abbilden des Inneren eines Untersuchungsobjekts (5), wobei:

    - das Untersuchungsobjekt (5) ausgehend von einer Strahlungsquellposition (15) mit Pulsen elektromagnetischer Strahlung (13) durchstrahlt wird, und
    - die durch das Untersuchungsobjekt (5) hindurchtretende Strahlung mittels einer Detektoranordnung (9) mit mehreren Detektoren (17) detektiert wird, wobei

    - die Detektoranordnung (9) derart betrieben wird, dass für jeden der Detektoren (17) die Strahlungsdetektion alternierend zu vorgegebenen Detektions-Anfangszeitpunkten ( $t_{DA,j}^{i}$ ) begonnen und zu vorgegebenen Detektions-Endzeitpunkten ( $t_{DE,j}^{i}$ ) beendet wird, wobei die Detektoranordnung (9) derart betrieben wird, dass zumindest einige der Detektoren (17.1, 17.2) mit unterschiedlichen Detektions-Endzeitpunkten ( $t_{DE,1}^{i}, t_{DE,2}^{i}$ ) angesteuert werden und unterschiedliche Detektions-Endzeitpunkte ( $t_{DE,1}^{i}, t_{DE,2}^{i}$ ) aufweisen, **dadurch gekennzeichnet, dass**

- die Detektoren (17.1, 17.2), welche unterschiedliche Detektions-Endzeitpunkte ( $t_{DE,1}^{i}, t_{DE,2}^{i}$ ) aufweisen, unterschiedliche Abstände zu der Strahlungsquellposition (15) aufweisen.

## Claims

1. Device (1) for transmission imaging for imaging the interior of an examination object (5), comprising:

   - a radiation generating device (7) for emitting pulses of electromagnetic radiation (13) for radiating through the examination object (5) proceeding from a radiation source position (15),
   - a detector arrangement (9) having a plurality of detectors (17) for detecting the radiation, and
   - a control device (11) designed for controlling the detector arrangement (9) in such a way that for each of the detectors (17) the radiation detection is alternately started at predefined detection start times $\left(t_{DA,j}^{i}\right)$ and ended at predefined detection end times $\left(t_{DE,j}^{i}\right)$, wherein the control device (11) is designed for controlling the detector arrangement (9) in such a way that at least some of the detectors (17.1, 17.2) are controlled with different detection end times $\left(t_{DE,1}^{i}, t_{DE,2}^{i}\right)$ and have different detection end times $\left(t_{DE,1}^{i}, t_{DE,2}^{i}\right)$, **characterized in that**

   - the detectors (17.1, 17.2) which have different detection end times $\left(t_{DE,1}^{i}, t_{DE,2}^{i}\right)$ are at different distances from the radiation source position (15).

2. Device according to Claim 1, wherein the control device (11) is designed for controlling the detector arrangement (9) in such a way that the detection end times $\left(t_{DE,2}^{i}\right)$ for detectors (17.2) at a larger distance from the radiation source position (15) are temporally after the detection end times $\left(t_{DE,1}^{i}\right)$ for detectors (17.1) at a, by comparison, smaller distance from the radiation source position (15).

3. Device according to either of Claims 1 and 2, wherein

   - the radiation generating device (7) is designed for emitting pulses of electromagnetic radiation (13) in such a way that the radiation emission emanating from the radiation source position (15) is alternately started at predefined pulse emission start times $\left(t_{PA}^{i}\right)$ and ended at predefined pulse emission end times $\left(t_{PE}^{i}\right)$, and
   - the control device (11) is designed for controlling the detector arrangement (9) in such a way that for each detector (17), between two successive pulse emission start times $\left(t_{PA}^{i}, t_{PA}^{i+1}\right)$, in each case a detection start time $\left(t_{DA,j}^{i+1}\right)$ and a detection end time $\left(t_{DE,j}^{i}\right)$ are present in such a way that the detection start time $\left(t_{DA,j}^{i+1}\right)$ is temporally after the detection end time $\left(t_{DE,j}^{i}\right)$.

4. Device according to any of Claims 1 to 3, wherein the control device (11) is designed for controlling the detector arrangement (9) in such a way that all detectors (17) have the same detection start times $\left(t_{DA,j}^{i}\right)$.

5. Device according to any of Claims 1 to 4, wherein the control device (11) is designed for controlling the detector arrangement (9) in such a way that the difference between the time interval present between a pulse emission start time $(t^i_{PA})$ and the subsequent detection end time $(t^i_{DE,j})$ of a respective detector and the time $(T^0_j)$, required to travel the distance between the radiation source position (15) and the respective detector at the speed of light is equal in magnitude for all detectors.

6. Device according to any of Claims 1 to 5, wherein the control device (11) is designed for controlling the detector arrangement (9) in such a way that for each detector (17) the time interval between a pulse emission start time $(t^i_{PA})$ and the subsequent detection end time $(t^i_{DE,j})$ is in each case smaller than the sum of the pulse duration $(T_P)$ and the time $(T^0_j)$, required to travel the distance between the radiation source position (15) and the respective detector (17) at the speed of light.

7. Device according to any of Claims 1 to 6, wherein the control device (11) is designed for controlling the detector arrangement (9) in such a way that for each detector the time interval between a pulse emission start time $(t^i_{PA})$ and the subsequent detection end time $(t^i_{DE,j})$ is less than double the time $(T^0_j)$, required to travel the distance between the radiation source position (15) and the respective detector (17) at the speed of light.

8. Device according to any of Claims 1 to 7, wherein the control device (11) is designed for controlling the detector arrangement (9) in such a way that for each detector (17) the time interval between a pulse emission end time $(t^i_{PE})$ and the subsequent detection start time $(t^{i+1}_{DA,j})$ is greater than double the time $(T^0_j)$, required to travel the distance between the radiation source position (15) and the respective detector (17) at the speed of light.

9. Device according to any of Claims 1 to 8, wherein the radiation generating device (7) is designed for generating pulses of electromagnetic radiation (13) which comprise or consist of photons having photon energies of at least 80 keV.

10. Method for transmission imaging for imaging the interior of an examination object (5), wherein:

- proceeding from a radiation source position (15) pulses of electromagnetic radiation (13) are radiated through the examination object (5), and
- the radiation passing through the examination object (5) is detected by means of a detector arrangement (9) having a plurality of detectors (17), wherein
- the detector arrangement (9) is operated in such a way that for each of the detectors (17) the radiation detection is alternately started at predefined detection start times $(t^i_{DA,j})$ and ended at predefined detection end times $(t^i_{DE,j})$, wherein the detector arrangement (9) is operated in such a way that at least some of the detectors (17.1, 17.2) are controlled with different detection end times $(t^i_{DE,1}, t^i_{DE,2})$ and have different detection end times $(t^i_{DE,1}, t^i_{DE,2})$, **characterized in that**
- the detectors (17.1, 17.2) which have different detection end times $(t^i_{DE,1}, t^i_{DE,2})$ are at different distances from the radiation source position (15).

**Revendications**

1. Dispositif (1) d'imagerie de transmission pour représenter l'intérieur d'un objet examiné (5), présentant :

- un dispositif de production de rayonnement (7) pour émettre des impulsions d'un rayonnement électromagnétique (13) pour radiographier l'objet examiné (5) à partir d'une position de source de rayonnement (15),
- un agencement de détecteur (9) pourvu de plusieurs détecteurs (17) pour détecter le rayonnement, et
- un dispositif de commande (11) qui est réalisé pour piloter l'agencement de détecteur (9) de telle sorte que pour chacun des détecteurs (17) la détection de rayonnement commence en alternance à des instants de début de détection $(t_{DA,j}^{i})$ prédéfinis et se termine à des instants de fin de détection $(t_{DE,j}^{i})$ prédéfinis, dans lequel le dispositif de commande (11) pour piloter l'agencement de détecteur (9) est réalisé de telle sorte qu'au moins certains des détecteurs (17.1, 17.2) sont pilotés avec différents instants de fin de détection $(t_{DE,1}^{i}, t_{DE,2}^{i})$ et présentent différents instants de fin de détection $(t_{DE,1}^{i}, t_{DE,2}^{i})$ , **caractérisé en ce que**
- les détecteurs (17.1, 17.2) qui présentent différents instants de fin de détection $(t_{DE,1}^{i}, t_{DE,2}^{i})$ présentent différentes distances par rapport à la position de source de rayonnement (15).

2. Dispositif selon la revendication 1, dans lequel le dispositif de commande (11) pour piloter l'agencement de détecteur (9) est réalisé de telle sorte que les instants de fin de détection $(t_{DE,2}^{i})$ pour les détecteurs (17.2) ayant une plus grande distance par rapport à la position de source de rayonnement (15) se trouvent dans le temps après les instants de fin de détection $(t_{DE,1}^{i})$ pour les détecteurs (17.1) ayant une distance inférieure par rapport à la position de source de rayonnement (15) en comparaison avec celle-ci.

3. Dispositif selon l'une quelconque des revendications 1 et 2, dans lequel

- le dispositif de production de rayonnement (7) pour émettre des impulsions d'un rayonnement électromagnétique (13) est réalisé de telle sorte que l'émission de rayonnement émanant de la position de source de rayonnement (15) commence en alternance à des instants de début d'émission d'impulsion prédéfinis $(t_{PA}^{i})$ et se termine à des instants de fin d'émission d'impulsion $(t_{PE}^{i})$ , et
- le dispositif de commande (11) pour piloter l'agencement de détecteur (9) est réalisé de telle sorte que pour chaque détecteur (17) entre deux instants de début d'émission d'impulsion $(t_{PA}^{i}, t_{PA}^{i+1})$ consécutifs, respectivement un instant de début de détection $(t_{DA,j}^{i+1})$ et un instant de fin de détection $(t_{DE,j}^{i})$ sont présents de telle sorte que l'instant de début de détection $(t_{DA,j}^{i+1})$ se trouve dans le temps après l'instant de fin de détection $(t_{DE,j}^{i})$ .

4. Dispositif selon l'une quelconque des revendications 1 à 3, dans lequel le dispositif de commande (11) pour piloter l'agencement de détecteur (9) est réalisé de telle sorte que tous les détecteurs (17) présentent les mêmes instants de début de détection $(t_{DA,j}^{i})$

5. Dispositif selon l'une quelconque des revendications 1 à 4, dans lequel le dispositif de commande (11) pour piloter l'agencement de détecteur (9) est réalisé de telle sorte que la différence entre l'intervalle de temps présent entre un instant de début d'émission d'impulsion $(t_{PA}^{i})$ et l'instant de fin de détection suivant $(t_{DE,j}^{i})$ d'un détecteur respectif, et le temps $(T_{j}^{0})$ , qui est nécessaire pour parcourir la distance entre la position de source de rayonnement (15) et le détecteur respectif à la vitesse de la lumière sont identiques pour tous les détecteurs.

6. Dispositif selon l'une quelconque des revendications 1 à 5, dans lequel le dispositif de commande (11) pour piloter l'agencement de détecteur (9) est réalisé de telle sorte que pour chaque détecteur (17) l'intervalle de temps entre un instant de début d'émission d'impulsion $(t_{PA}^i)$ et l'instant de fin de détection $(t_{DE,j}^i)$ suivant, est respectivement inférieur à la somme de la durée d'impulsion ($T_P$) et du temps $(T_j^0)$, qui est nécessaire pour parcourir la distance entre la position de source de rayonnement (15) et le détecteur (17) respectif à la vitesse de la lumière.

7. Dispositif selon l'une quelconque des revendications 1 à 6, dans lequel le dispositif de commande (11) pour piloter l'agencement de détecteur (9) est réalisé de telle sorte que pour chaque détecteur l'intervalle de temps entre un instant de début d'émission d'impulsion $(t_{PA}^i)$ et l'instant de fin de détection $(t_{DE,j}^i)$ suivant est inférieur au double du temps $(T_j^0)$, qui est nécessaire pour parcourir la distance entre la position de source de rayonnement (15) et le détecteur (17) respectif à la vitesse de la lumière.

8. Dispositif selon l'une quelconque des revendications 1 à 7, dans lequel le dispositif de commande (11) pour piloter l'agencement de détecteur (9) est réalisé de telle sorte que pour chaque détecteur (17) l'intervalle de temps entre un instant de fin d'émission d'impulsion ( $t_{PE}^i$ ) et l'instant de début de détection ( $t_{DA,j}^{i+1}$ ) suivant est supérieur au double du temps ( $T_j^0$ ), qui est nécessaire pour parcourir la distance entre la position de source de rayonnement (15) et le détecteur (17) respectif à la vitesse de la lumière.

9. Dispositif selon l'une quelconque des revendications 1 à 8, dans lequel le dispositif de production de rayonnement (7) est réalisé pour produire des impulsions d'un rayonnement électromagnétique (13) qui présentent des photons ayant des énergies photoniques d'au moins 80 keV ou en sont composées.

10. Procédé d'imagerie de transmission pour représenter l'intérieur d'un objet examiné (5), dans lequel :

- l'objet examiné (5) est radiographié en partant d'une position de source de rayonnement (15) avec des impulsions d'un rayonnement électromagnétique (13), et
- le rayonnement traversant l'objet examiné (5) est détecté au moyen d'un agencement de détecteur (9) pourvu de plusieurs détecteurs (17), dans lequel
- l'agencement de détecteur (9) fonctionne de telle sorte que pour chacun des détecteurs (17) la détection de rayonnement commence en alternance à des instants de début de détection ( $t_{DA,j}^i$ ) prédéfinis et se termine à des instants de fin de détection ( $t_{DE,j}^i$ ) prédéfinis, dans lequel l'agencement de détecteur (9) fonctionne de telle sorte qu'au moins certains des détecteurs (17.1, 17.2) sont pilotés avec différents instants de fin de détection ( $t_{DE,1}^i$, $t_{DE,2}^i$ ) et présentent différents instants de fin de détection ( $t_{DE,1}^i$, $t_{DE,2}^i$ ), **caractérisé en ce que**

- les détecteurs (17.1, 17.2) qui présentent différents instants de fin de détection ( $t_{DE,1}^i$, $t_{DE,2}^i$ ) présentent différentes distances par rapport à la position de source de rayonnement (15).

Figur 1

Figur 1A

EP 4 139 666 B1

Figur 2

Figur 3

Figur 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2006008047 A1